# EUROPEAN PATENT SPECIFICATION

(11) **EP 4 182 395 B1**
(45) Date of publication and mention of the grant of the patent: **09.09.2026**
(21) Application number: 21740524.0
(22) Date of filing: 09.07.2021
(51) Int. Cl.: C09D 11/037, C09D 11/50, B33Y 70/00, B41M 3/14, C07D 233/84, C07D 239/22

(54) **DITHIOLENE METAL COMPLEXES**
DITHIOLEN-METALL-KOMPLEXE
COMPLEXES MÉTALLIQUES DE DITHIOLÈNE

(30) Priority: 16.07.2020 EP 20186317
(43) Date of publication of application: 24.05.2023
(73) Proprietor: BASF SE, 67056 Ludwigshafen am Rhein (DE)
(72) Inventor: REICHERT, Hans, 4057 Basel (CH); SEEGER, Oliver, 67056 Ludwigshafen (DE); REICHELT, Helmut, 67056 Ludwigshafen (DE); DORMANN, Korinna, 67056 Ludwigshafen (DE); LEYBACH, Holger, 4057 Basel (CH)
(74) Representative: BASF IP Association
(86) International application number: PCT/EP2021/069111
(87) International publication number: WO 2022/013081

(56) References cited:
- EP-A1- 3 067 216
- WO-A1-2012/069518
- WO-A1-2020/079154
- JP-A- 2003 262 953
- JP-A- 2004 045 653

## Description

**The present** invention relates to particles of a compound of formula (I), wherein the particles have a median particle size (D₅₀) in the range of from 30 nm to 90 nm, preferably from 40 nm to 80 nm, more preferably 50 to 70 nm with D10 being greater than 20 nm, especially greater than 25 nm, very especially greater than 30 nm, their use as almost colourless IR absorbers, for optical filter applications, especially for plasma display panels, or for laser welding of plastics. The compounds may be used in compositions for inks, paints and plastics, especially in a wide variety of printing systems and are particularly well-suited for security applications.

### Description of the related art

Colourless, or at least barely coloured, IR absorbers meet a significant technical need in a wide range of applications, such as security printing (bank notes, credit cards, identity cards, passports etc.), invisible and/or IR readable bar codes, the laser-welding of plastics, the curing of surface-coatings using IR radiators, the drying and curing of print, the fixing of toners on paper or plastics, optical filters for PDPs (plasma display panels), laser marking e.g. of paper or plastics, the heating of plastics preforms, heat shielding applications, fusing agent for 3D printing, fuel marking, marker for circular economic products etc.

A large number of organic and inorganic substances belonging to different compound classes and with a great variety of different structures are known for the application as IR absorbers. Notwithstanding that large numbers of known compound classes and structures, the provision of products with a complex profile of properties often presents difficulties. There is a continuing demand for IR absorber that are "colourlessness" (i.e. with the minimum possible inherent colour), and that simultaneously meet the technical stability requirements (chemical stability, heat stability and/or light stability).

A special field of application for colourless IR absorbers regards inks for printing processes which are used for printing currency and other security documents, also referred to as "security printing". Typical security printing processes are processes, wherein an ink composition is employed that is designed to selectively absorb radiation in parts of the "optical infrared" spectrum, whilst being transparent in other parts of it. IR absorbers for security printing are available, for example, from "American Dye Source", but virtually all of them have a noticeable absorption in the VIS range of the spectrum (from 400 to 700 nm).

US2008/0241492 describes an intaglio printing ink for a security printing process, wherein the ink comprises a polymeric organic binder and an infrared absorbing material that comprises transition element atoms or ions whose infrared absorption is a consequence of electronic transitions within the d-shell of the transition element. Suitable transition elements are Ti, V, Cr, Mn, Fe, Co, Ni, and Cu. In a suitable embodiment, the infrared absorbing material is a glass, in which there is a coordination of the transition element ions to phosphate and/or fluoride anions present in the glass. In a further suitable embodiment, the infrared absorbing material is an IR-absorbing transition element atom or ion bound to the polymer binder of the ink. In particular, the infrared absorbing material is an IR-absorbing complex of a transition element atom or ion and a binding site contained in the polymer, e.g. an organic thiourea-copper(II) complex dissolved in the polymeric binder.

US5,282,894 describes a liquid useful as printing ink that contains one or more dyes with their absorption maximum within the range from 700 to 1200 nm selected from phthalocyanines, naphthalocyanines, nickel-dithiolene complexes, aminium compounds of aromatic amines, methine dyes or azulenesquaric acid dyes, as well as solvent and binder.

WO2007/091094 describes an image article that comprises a substrate having a security image coated on at least a portion thereof, wherein the security image comprises a defined infrared-absorbing compound, for example Pigment Green 8, that does not create a strongly coloured security image. The disclosed infrared-absorbing compounds still have a noticeable absorption in the VIS range of the spectrum.

WO2007/132214 describes a composition comprising an ink and an infrared-absorbing material that comprises a metal, a metal salt, a metal oxide or metal nitride, wherein the metal is in particular selected from periods 4, 5 or the lanthanides. Also described is an article comprising a substrate having imaged thereon an infrared-absorbing material to form a security image, and a method of manufacture of such an article by imagewise application of a composition comprising such an infrared-absorbing material to a substrate.

M. Arca et al. describe in J. Chem. Soc., Dalton Trans. 1998, 3731-3736 metal dithiolenes (see scheme 1) belonging to the general class [M(R,R'timdt)₂] (M = Ni, Pd; (R,R'timdt) = monoanion of disubstituted imidazolidine-2,4,5-trithione; R and R' = ethyl or isopropyl). As those metal dithiolenes exhibit large π delocalization they can also be characterized by the aromatic resonance structure on the right:

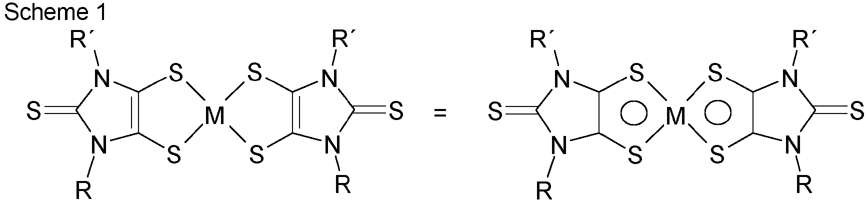

JP2003262953A, JP2004045653A, JP200599755A and WO2020159407 describe metal dithiolenes [M(R,R'timdt)₂], wherein R and R' are selected from unsubstituted and substituted alkyl, cycloalkyl and aryl groups.

M.C. Aragoni et al. describe in Eur. J. Inorg. Chem. 2003, 1939-1947 NIR dyes based on [M(R,R'timdt)₂] metal dithiolenes, wherein R and R' are inter alia selected from unsubstituted and substituted aryl groups.

WO2008/086931 teaches the use of dithiolene metal complexes [M(L)₂], wherein L is the monoanion of a disubstituted imidazolidine-2-chalcogenone-4,5-dithione and the chalcogene is O or S, as colourless IR absorbers. Whereas aryl substituted compounds are mentioned in very general terms, there is no concrete teaching with regard to those compounds. In particular, in all examples the nitrogen atoms bear only unsubstituted and substituted alkyl and alkenyl groups.

US20040207700 relates to a stabilized ink composition comprising an IR-absorbing metal-dithiolene dye and a singlet oxygen quencher. The singlet oxygen quencher is selected from ascorbic acid, 1,4-diazabicyclo-[2.2.2]octane (DABCO), azides (e.g. sodium azide), histidine or tryptophan.

WO2012069518 relates to the use of specific metal complexes of dithiolenes with aryl or heteroaryl substituted imidazolidine-2-chalcogenone-4,5-dithione ligands as colourless IR absorbers.

WO2012152584 relates to specific metal complexes of dithiolenes with perfluoroalkyl substituted imidazolidine-2-chalcogenone-4,5-dithione ligands, a process for their preparation and their use as colourless IR absorbers, for optical filters application; especially for plasma display panels, or for laser welding of plastics.

EP2942378 relates to an inkjet ink composition comprising: at least one near infrared (NIR) absorbent selected from the group consisting of: metal dithiolene complexes, cyanines, and phthalocyanines in amount of from 0,05% to 5% w/w; gamma-butyrolactone as a solvent in amount of from 1% to 30% w/w; 2-butoxyethyl acetate as a solvent in amount of from 50% to 95% w/w; and vinylchloride-vinylacetate copolymer as a binder in amount of from 0.1% to 5% w/w.

EP3067216 relates to chromophoric compositions, in particular to compositions containing as chromophore an IR absorbing compound and specific stabilizing compounds which prevent the oxidative degradation of the chromophore.

EP3078503A1 relates to a security document including a transparent window, wherein an infrared absorbing material is applied to or incorporated within the window, wherein the infrared absorbing material comprises an infrared absorbing dye, and infrared **absorbing** nanoparticles having an average size of not greater than 100nm, and wherein the infrared absorbing material is substantially transparent to visible radiation. The infrared absorbing dye is an organic or complexed dye, or a perylene-based or dithiolene-based dye, or is selected from a group including Lumogen 765 and Lumogen 788.

WO2019/057683 describes a new crystal form of bis(diphenylimidazolidinetrithione-κS4, κS5)-, (SP-4-1)-nickel(II).

WO2020/079154 relates to microparticle compositions comprising an organic IR absorbing pigment having a main absorption maximum in the range from 750 to 1100 nm. The particle size distribution of the particles of the IR absorbing organic pigment is typically characterized by having a particle size, which is smaller than the particle size of the microparticles containing the solid particles of the IR-absorbing organic pigment, which are surrounded or embedded by an aminoplast polymer, e.g. by having a D(v, 0.5) in the range from 10 to 800 nm, in particular from 20 to 500 nm, more particularly in the range from 20 to 300 nm, as determined by static light scattering.

### Description of the invention

The objective of the present invention is to provide (dithiolene) metal complexes [M(L)₂] with absorbing properties, light stability and heat stability as high as possible. Supris-ingly it has been found that by kneading dithiolene metal complexes particles can be obtained having improved absorbing properties and stability, e.g. better resistance to the attack of chemicals, especially radicals in offset inks.

Accordingly, the present invention relates to particles of the compound of formula (I) having a median particle size (D₅₀) in the range of from 30 nm to 90 nm, preferably from 40 nm to 80 nm, more preferably 50 to 70 nm with D₁₀ being greater than 20 nm, especially greater than 25 nm, very especially greater than 30 nm. The compounds exhibit high absorbing properties, thermal and light fastness, high resistance against chemicals and solvents without losing their other advantages like colourlessness. They can be advantageously employed as IR absorbers for security printing and the laser-welding of plastics. Due to their unique application properties they are in particular suitable as IR absorbers for security printing, especially for value documents, ID cards, tax stamps and bank notes.

In a first aspect, the invention relates to particles of a compound of formula 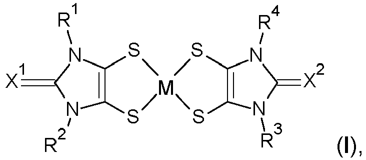 wherein
M is aelected from Ni, Pd and Pt,
X¹ and X² are each independently of each other sulfur or oxygen,
R¹, R², R³, and R⁴ are independently selected from an unsubstituted, or substituted alkyl group, an unsubstituted, or substituted cycloalkyl group, an unsubstituted or substituted aryl group and an unsubstituted or substituted heteroaryl group,
wherein the particles have a median particle size (D₅₀) in the range of from 30 nm to 90 nm.

Preferably, the particles have a median particle size (D₅₀) in the range of from 40 nm to 80 nm, more preferably in the range of 50 to 70 nm.

Preferably, the particles have a D₁₀ of greater than 20 nm, more preferably of greater than 25 nm and most preferred of greater than 30 nm.

In a preferred embodiment the particles of the compound of formula (I), especially of formula (la) and (Ib), have a D₅₀ in the range of from 40 to 80 nm with D₁₀ being greater than 25 nm.

In a more preferred embodiment the particles of the compound of formula (I), especially of formula (la) and (Ib), have a D₅₀ in the range of from 50 to 70 nm with D₁₀ being greater than 30 nm.

The particles have usually a number average particle size in the range of from 35 nm to 95 nm, preferably from 45 nm to 85 nm, more preferably 55 to 75 nm with standard deviation being less than 45 nm, especially less than 40 nm, very especially less than 35 nm.

The particles of the compounds of formula (I) may be used as colourless IR absorber, for optical filter applications, for plasma display panels, for laser marking of paper or plastics, for laser welding of plastics, for 3D printing, for fuel marking, as marker for circular economic products, for the curing of surface-coatings using IR radiators, for the drying and curing of print, for the fixing of toners on paper or plastics, for heat shielding applications, for invisible and/or IR readable bar codes, or as IR absorber in security printing.

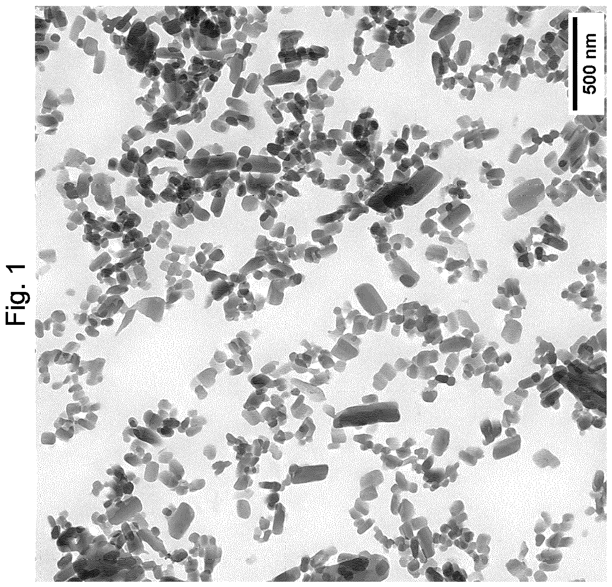

Fig. 1 is a Transmission Electron Micrograph (TEM) of the particles of the compound 1, obtained in Example 1.

Fig. 2: Absorption (100 - % Remission) of compound 1 and compound CC-1 at lambda max. Absorption of compound 1 at 900 nm is set to 100 %.

The number average particle size is the number weighted mean diameter (Feret diameter). The median particle size (D₅₀) is the value separating the higher half of the data from the lower half. It is the determined particle size from which half of the particles are smaller and half are larger. D₁₀: The portion of particles with diameters below this value is 10%.

Preferably, in the compounds of the general formula (I) M is Ni, or Pt. In particular, in the compounds of the general formula (I) M is Ni.

The compound of formula (I) is preferably a compound of formula 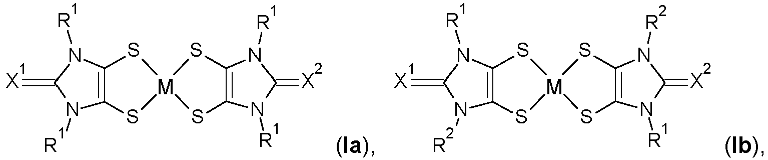 or 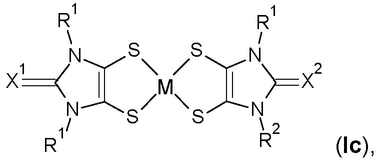 wherein M, X¹, X², R¹ and R² are defined above, or below.

Compounds of formula (la) and (Ib) are preferred and compounds of formula (la) are most preferred.

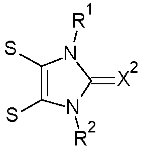

Metal complexes [M(L)₂], wherein L is (R¹ is different from R²) differ in the relative orientation of substituents and can be present as geometric isomers: 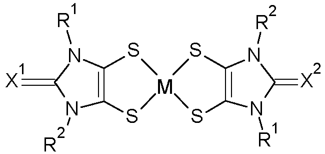 (trans) and 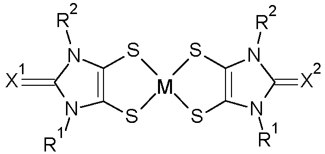 (cis).

For simplification, both isomers are represented throughout the present application by formula

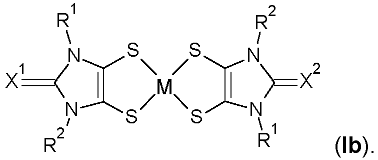

X¹ and X² may be oxygen, or X¹ is oxygen and X² is sulfur. Preferably X¹ and X² are sulfur.

In a preferred embodiment the invention is directed to particles of the compound of formula (la), wherein R' is an unsubstituted, or substituted alkyl group, an unsubstituted or substituted aryl group.

In another preferred embodiment the invention is directed to the compound of formula (Ib), or (Ic), wherein R' is an unsubstituted, or substituted alkyl group and R² an unsubstituted or substituted aryl group.

M is Ni, Pd or Pt, especially Ni, or Pt, very especially Ni.

The invention also relates to particles of compounds of the general formula (I), wherein X¹ is oxygen and X² is sulfur or oxygen.

In the context of the invention, the expression "halogen" denotes in each case fluorine, bromine, chlorine or iodine, preferably fluorine, chlorine or bromine, in particular fluorine or chlorine.

In the context of the present invention and with regard to the substituents R¹, R², R³, and R⁴, the expression "alkyl" comprises straight-chain or branched alkyl groups. Alkyl is preferably C₁-C₃₀-alkyl, more preferably C₁-C₂₀-alkyl, most preferably C₁-C₁₂-alkyl, in particular C₁-C₆-alkyl. Examples of alkyl groups are especially methyl, ethyl, n-propyl, isopropyl, n-butyl, isobutyl, sec-butyl, tert-butyl, n-pentyl, neo-pentyl, n-hexyl, n-heptyl, n-octyl, n-nonyl, n-decyl, n-undecyl, n-dodecyl, n-tridecyl, n-tetradecyl, n-hexadecyl, n-octadecyl and n-eicosyl.

The expression alkyl also comprises alkyl radicals whose carbon chains may be interrupted by one or more groups which are independently selected from -O- and -S-.

Substituted alkyl groups may, depending on the length of the alkyl chain, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, COOH, carboxylate, alkylcarbonyloxy, carbamoyl, alkylaminocarbonyl, (dialkylamino)carbonyl, SO₃H, sulfonate, sulfoamino, sulfamide, sulfamoyl, amidino, NE¹E² where E¹ and E² are each independently hydrogen, alkyl, cycloalkyl, heterocycloalkyl, aryl or heteroaryl. Cycloalkyl, heterocycloalkyl, aryl and heteroaryl substituents of the alkyl groups may in turn be unsubstituted or substituted; suitable substituents are the substituents mentioned below for these groups.

The expression substituted alkyl group also comprises alkyl radicals that have one or more (e.g. 1, 2, 3, 4, 5 or more than 5) substituents and whose carbon chains may be interrupted by one or more groups which are independently selected from -O- and -S-. Carboxylate and sulfonate respectively represent a metal carboxylate or metal sulfonate, or a carboxylic ester function or sulfonic ester function.

The above remarks regarding alkyl also apply to the alkyl moiety in alkoxy, alkylthio (= alkylsulfanyl), monoalkylamino and dialkylamino.

In the context of the present invention and with regard to the substituents R¹, R², R³, and R⁴, the term "cycloalkyl" denotes a mono-, bi- or tricyclic hydrocarbon radical having usually from 3 to 20, preferably 3 to 12, more preferably 5 to 12, carbon atoms such as cyclopropyl, cyclobutyl, cyclopentyl, cyclohexyl, cycloheptyl, cyclooctyl, cyclododecyl, cyclopentadecyl, norbornyl, bicyclo[2.2.2]octyl or adamantyl.

Substituted cycloalkyl groups may, depending on the ring size, have one or more (e.g. 1, 2, 3, 4, 5 or more than 5) substituents. These are preferably each independently selected from alkyl, alkoxy, alkylthio, cycloalkyl, heterocycloalkyl, aryl, heteroaryl, fluorine, chlorine, bromine, hydroxyl, mercapto, cyano, nitro, nitroso, formyl, acyl, COOH, carboxylate, alkylcarbonyloxy, carbamoyl, SO₃H, sulfonate, sulfamino, sulfamide, amidino, NE³E⁴ where E³ and E⁴ are each independently hydrogen, alkyl, cycloalkyl, heterocycloalkyl, aryl or heteroaryl. In the case of substitution, the cycloalkyl groups preferably bear one or more, for example one, two, three, four or five, C₁-C₆-alkyl groups. Examples of substituted cycloalkyl groups are especially 2- and 3-methylcyclopentyl, 2- and 3-ethylcyclopentyl, 2-, 3- and 4-methylcyclohexyl, 2-, 3- and 4-ethylcyclohexyl, 2-, 3-and 4-propylcyclohexyl, 2-, 3- and 4-isopropylcyclohexyl, 2-, 3- and 4-butylcyclohexyl, 2-, 3- and 4-sec.-butylcyclohexyl, 2-, 3- and 4-tert-butylcyclohexyl, 2-, 3- and 4-methyl-cycloheptyl, 2-, 3- and 4-ethylcycloheptyl, 2-, 3- and 4-propylcycloheptyl, 2-, 3- and 4-isopropylcycloheptyl, 2-, 3- and 4-butylcycloheptyl, 2-, 3- and 4-sec-butylcycloheptyl, 2-, 3- and 4-tert-butylcycloheptyl, 2-, 3-, 4- and 5-methylcyclooctyl, 2-, 3-, 4- and 5-ethyl-cyclooctyl, 2-, 3-, 4- and 5-propylcyclooctyl.

The above remarks regarding cycloalkyl also apply to the cycloalkyl moiety in cycloalkoxy, cycloalkylthio (= cycloalkylsulfanyl), monocycloalkylamino and dicycloalkylamino.

In the context of the present invention, the expression "heterocycloalkyl" comprises nonaromatic, unsaturated or fully saturated, cycloaliphatic groups having generally 5 to 8 ring atoms, preferably 5 or 6 ring atoms. In the heterocycloalkyl groups, compared to the corresponding cycloalkyl groups, 1, 2, 3, 4 or more than 4 of the ring carbon atoms are replaced by heteroatoms or heteroatom-containing groups. The heteroatoms or heteroatom-containing groups are preferably selected from -O-, -S-, -NR^{a}-, -C(=O)-, -S(=O)- and/or -S(=O)₂-. R^{a} is preferably hydrogen, alkyl, cycloalkyl, heterocycloalkyl, aryl or heteroaryl. Examples of heterocycloalkyl groups are especially pyrrolidinyl, piperidinyl, imidazolidinyl, pyrazolidinyl, oxazolidinyl, morpholinyl, thiazolidinyl, isothiazolidinyl, isoxazolidinyl, piperazinyl, tetrahydrothiophenyl, dihydrothien-2-yl, **tetrahydrofuranyl,** dihydrofuran-2-yl, tetrahydropyranyl, 2-oxazolinyl, 3-oxazolinyl, 4-oxazolinyl and dioxanyl.

Substituted heterocycloalkyl groups may, depending on the ring size, have one or more (e.g. 1, 2, 3, 4, 5 or more than 5) substituents. These are preferably each independently selected from alkyl, alkoxy, alkylthio, cycloalkyl, heterocycloalkyl, aryl, heteroaryl, fluorine, chlorine, bromine, hydroxyl, mercapto, cyano, nitro, nitroso, formyl, acyl, COOH, carboxylate, alkylcarbonyloxy, carbamoyl, alkylaminocarbonyl, (dialkylamino)carbonyl, SO₃H, sulfonate, sulfoamino, sulfamide, sulfamoyl, amidino, NE⁵E⁶ where E⁵ and E⁶ are each independently hydrogen, alkyl, cycloalkyl, heterocycloalkyl, aryl or heteroaryl. In the case of substitution, the heterocycloalkyl groups preferably bear one or more, for example one, two, three, four or five, C₁-C₆-alkyl groups.

The above remarks regarding heterocycloalkyl also apply to the heterocycloalkyl moiety in heterocycloalkoxy, heterocycloalkylthio (= heterocycloalkylsulfanyl), (monoheter-ocycloalkyl)amino and (diheterocycloalkyl)amino.

In the context of the present invention, the term "aryl" refers to mono- or polycyclic aromatic hydrocarbon radicals. Suitable and preferred unsubstituted and substituted aryl groups are defined in the following with regard to the substituents R¹, R², R³, and R⁴.

In the context of the present invention, the term "heteroaryl" (hetaryl) refers to unsubstituted or substituted heteroaromatic, mono- or polycyclic groups. Suitable and preferred unsubstituted and substituted heteroaryl groups are defined in the following with regard to the substituents R¹, R², R³, and R⁴.

According to the invention, R¹, R², R³, and R⁴ are independently selected from unsubstituted or substituted aryl and unsubstituted or substituted heteroaryl.

The unsubstituted or substituted aryl groups R¹, R², R³, and R⁴ are independently selected from unsubstituted or substituted mono- or polycyclic aromatic hydrocarbon radicals, preferably having 6 to 24 carbon atoms, more preferably having 6 to 20 carbon atoms, especially having 6 to 14 carbon atoms as ring members.

The unsubstituted or substituted aryl groups R¹, R², R³, and R⁴ are preferably selected from unsubstituted or substituted phenyl, unsubstituted or substituted naphthyl, unsubstituted or substituted indenyl, unsubstituted or substituted fluorenyl, unsubstituted or substituted anthracenyl, unsubstituted or substituted phenanthrenyl, unsubstituted or substituted naphthacenyl, unsubstituted or substituted chrysenyl, unsubstituted or substituted pyrenyl, unsubstituted or substituted coronenyl and unsubstituted or substituted perylenyl.

The unsubstituted or substituted aryl groups R¹, R², R³, and R⁴ are more preferably selected from unsubstituted or substituted phenyl and unsubstituted or substituted naphthyl.

The unsubstituted or substituted aryl groups R¹, R², R³, and R⁴ are in particular selected from unsubstituted or substituted phenyl.

In a preferred embodiment, at least one of groups R¹, R², R³, and R⁴ is unsubstituted phenyl. In a particular preferred embodiment, all of groups R¹, R², R³, and R⁴ are unsubstituted phenyl.

The substituted aryl groups R¹, R², R³, and R⁴ may, depending on the number and size of their ring systems, have one or more (e.g. 1, 2, 3, 4, 5 or more than 5) substituents. The substituents of the substituted aryl groups R¹, R², R³, and R⁴ are preferably each independently selected from alkyl, alkoxy, alkylthio, cycloalkyl, heterocycloalkyl, aryl, heteroaryl, fluorine, chlorine, bromine, hydroxyl, mercapto, cyano, nitro, nitroso, formyl, acyl, COOH, carboxylate, alkylcarbonyloxy, carbamoyl, SO₃H, sulfonate, sulfamino, sulfamide, amidino, NE¹E² where E¹ and E² are each independently hydrogen, alkyl, cycloalkyl, heterocycloalkyl, aryl or heteroaryl. The alkyl, alkoxy, alkylamino, alkylthio, cycloalkyl, heterocycloalkyl, aryl and heteroaryl substituents on the substituted aryl groups R¹, R², R³, and R⁴ may in turn be unsubstituted or substituted. Reference is made to the substituents mentioned for these groups above and in the following.

The substituents on the substituted aryl groups R¹, R², R³, and R⁴ are preferably selected from alkyl; alkoxy; alkyl or alkoxy whose carbon chain is interrupted by one or more nonadjacent groups selected from -O-, -S-, -NR^{a}-, -C(=O)-, -S(=O)- and/or -S(=O)₂-, wherein R^{a} is hydrogen, alkyl, cycloalkyl, heterocycloalkyl, aryl or heteroaryl; haloalkyl; haloalkoxy; cycloalkyl; fluorine; chlorine; bromine; cyano and nitro.

The substituted aryl groups R¹, R², R³, and R⁴ are preferably substituted phenyl which bears 1, 2, 3, 4 or 5 substituents. The substituted aryl groups R¹, R², R³, and R⁴ are more preferably substituted phenyl which bears preferably 1, 2 or 3 substituents.

The substituted aryl groups R¹, R², R³, and R⁴ are preferably selected from aryl groups substituted by at least one alkyl group ("alkaryl", also referred to as alkylaryl). Alkaryl groups may, depending on the size of the aromatic ring system, have one or more (e.g. 1, 2, 3, 4, 5, 6, 7, 8, 9 or more than 9) alkyl substituents. The alkyl substituents on the alkaryl groups may be unsubstituted or substituted. In this regard, reference is made to the above statements regarding unsubstituted and substituted alkyl. In a preferred embodiment, the alkaryl groups have exclusively unsubstituted alkyl substituents. Alkaryl is preferably phenyl which bears 1, 2, 3, 4 or 5, preferably 1, 2 or 3, more preferably 1 or 2, alkyl substituents. The alkyl substituents on the alkaryl groups R¹, R², R³, and R⁴ are preferably selected from C₁-C₂₀-alkyl, more preferably C₁-C₁₂-alkyl and most preferably C₁-C₆-alkyl. Examples of alkyl groups are especially methyl, ethyl, n-propyl, isopropyl, n-butyl, isobutyl, sec-butyl, tert-butyl, n-pentyl, neo-pentyl, n-hexyl, n-heptyl, n-octyl, n-nonyl, n-decyl, n-undecyl, n-dodecyl, n-tridecyl, n-tetradecyl, n-hexadecyl, n-octadecyl and n-eicosyl.

The substituted aryl groups R¹, R², R³, and R⁴ are preferably selected from 2-, 3- and 4-methylphenyl, 2,4-, 2,5-, 3,5- and 2,6-dimethylphenyl, 2,4,6-trimethylphenyl, 2-, 3- and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl and 2,4,6-tri-tert-butylphenyl; 2-, 3- and 4-methoxyphenyl, 2,4-, 2,5-, 3,5- and 2,6-dimethoxyphenyl, 2,4,6-trimethoxyphenyl, 2-, 3- and 4-ethoxyphenyl, 2,4-, 2,5-, 3,5- and 2,6-diethoxyphenyl, 2,4,6-triethoxyphenyl, 2-, 3- and 4-propoxyphenyl, 2,4-, 2,5-, 3,5- and 2,6-dipropoxyphenyl, 2-, 3- and 4-isopropoxyphenyl, 2,4-, 2,5-, 3,5- and 2,6-diisopropoxyphenyl and 2-, 3- and 4-butoxyphenyl, 2-, 3- and 4-fluorophenyl, 2-, 3- and 4-chlorophenyl, (2-chloro-6-methyl)phenyl, (2-chloro-6-ethyl)phenyl, (4-chloro-6-methyl)phenyl, (4-chloro-6-ethyl)phenyl, (2-fluoro-6-methyl)phenyl, (2-fluoro-6-ethyl)phenyl, (4-fluoro-6-methyl)phenyl, (4-fluoro-6-ethyl)phenyl, pentafluorophenyl, pentachlorophenyl, 2,3,5,6-tetrafluorophenyl, 2,3,5,6-tetrachlorophenyl, 2,4-difluorophenyl, 2,4,5-trifluorophenyl, 2,4,6-trifluorophenyl, 2,5-difluorophenyl, 2-fluoro-5-(trifluoromethyl)phenyl, 2-fluoro-5-methylphenyl, 2,6-difluorophenyl, 4-chloro-2-fluorophenyl, 2,3,4-trifluorophenyl, 2,3,6-trifluorophenyl, 2,3,4,5-tetrafluorophenyl, 2-chloro-6-fluorophenyl, 2-fluoro-4-methylphenyl, 3-chloro-2,4-difluorophenyl, 3,5-dichloro-2,4-difluorophenyl, 1-chloro-4-fluorophenyl, 2-fluoro-3-(trifluoromethyl)phenyl, 2-(trifluoromethyl)-6-fluorophenyl, 2,3,4,6-tetrafluorophenyl, 3-chloro-2-fluorophenyl, 5-chloro-2-fluorophenyl, 2,3,5-trifluorophenyl, 4-(trifluoromethyl)-2,3,5,6-tetrafluorophenyl, 2-chloro-4,6-difluorophenyl, 2,5-difluoro-4-(trifluoromethyl)phenyl, 4-trifluoromethyl-2,3-difluorophenyl, 2,4-difluoro-3-(trifluoromethyl)phenyl, 2-fluoro-4-(trifluoromethyl)phenyl, 2-fluoro-5-methylsulphonylpenyl, 2-fluoro-4-hydroxymethylphenyl, 4-(trifluoromethyl)phenyl, 2-chloro-4-(trifluoromethyl)phenyl, 2,6-di-chloro-4-(trifluoromethyl)phenyl, 4-(trifluoromethyl)-2,3,5,6-tetrafluorophenyl, 3-fluoro-4-(trifluoromethyl)phenyl, 2,5-difluoro-4-(trifluoromethyl)phenyl, 3,5-difluoro-4-(trifluoromethyl)phenyl, 2,3-difluoro-4-(trifluoromethyl)phenyl, 2,4-bis(trifluoromethyl)phenyl, 3-chloro-4-(trifluoromethyl)phenyl, 2,4,6-tris(trifluoromethyl)phenyl, 3,4-bis(trifluoromethyl)phenyl, 2-fluoro-3-(trifluoromethyl)phenyl, 2-methyl-4-(trifluoromethyl)phenyl, 3,5-dichloro-4-(trifluoromethyl)phenyl, 2,3,6-trichloro-4-(trifluoromethyl)phenyl, etc.

The unsubstituted or substituted heteroaryl groups R¹, R², R³, and R⁴ are independently selected from unsubstituted or substituted heteroaromatic, mono- or polycyclic groups. In addition to the ring carbon atoms, these have 1, 2, 3, 4 or more than 4 heteroatoms as ring members. The heteroatoms are preferably selected from oxygen, nitrogen, selenium and sulfur. The heteroaryl groups have preferably 5 to 18, e.g. 5, 6, 8, 9, 10, 11, 12, 13 or 14, ring atoms.

Unsubstituted or substituted monocyclic heteroaryl groups R¹, R², R³, and R⁴ are preferably selected from unsubstituted or substituted 5- or 6-membered heteroaryl groups, such as 2-furyl (furan-2-yl), 3-furyl (furan-3-yl), 2-thienyl (thiophen-2-yl), 3-thienyl (thio-phen-3-yl), selenophen-2-yl, selenophen-3-yl, 1H-pyrrol-2-yl, 1H-pyrrol-3-yl, pyrrol-1-yl, imidazol-2-yl, imidazol-1-yl, imidazol-4-yl, pyrazol-1-yl, pyrazol-3-yl, pyrazol-4-yl, pyra-zol-5-yl, 3-isoxazolyl, 4-isoxazolyl, 5-isoxazolyl, 3-isothiazolyl, 4-isothiazolyl, 5-isothiazolyl, 2-oxazolyl, 4-oxazolyl, 5-oxazolyl, 2-thiazolyl, 4-thiazolyl, 5-thiazolyl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 4H-[1,2,4]-triazol-3-yl, 1,3,4-triazol-2-yl, 1,2,3-triazol-1-yl, 1,2,4-triazol-1-yl, pyridin-2-yl, pyridin-3-yl, pyridin-4-yl, 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

Unsubstituted or substituted polycyclic heteroaryl groups R¹, R², R³, and R⁴ preferably have 2, 3, 4 or more than 4 fused rings. The fused-on rings may be aromatic, saturated or partly unsaturated. Examples of polycyclic heteroaryl groups R¹, R², R³, and R⁴ are quinolinyl, isoquinolinyl, indolyl, isoindolyl, indolizinyl, benzofuranyl, isobenzofuranyl, benzothiophenyl, benzoxazolyl, benzisoxazolyl, benzthiazolyl, benzoxadiazolyl, benzothiadiazolyl, benzoxazinyl, benzopyrazolyl, benzimidazolyl, benzotriazolyl, benzotriazinyl, benzoselenophenyl, thienothiophenyl, thienopyrimidyl, thiazolothiazolyl, dibenzopyrrolyl (carbazolyl), dibenzofuranyl, dibenzothiophenyl, naphtho[2,3-b]thiophenyl, naphtha[2,3-b]furyl, dihydroindolyl, dihydroindolizinyl, dihydroisoindolyl, dihydroquinolinyl and dihydroisoquinolinyl.

The substituted hetaryl groups R¹, R², R³, and R⁴ may, depending on the number and size of their ring systems, have one or more (e.g. 1, 2, 3, 4, 5 or more than 5) substituents. These are preferably each independently selected from alkyl, alkoxy, alkylthio, cycloalkyl, heterocycloalkyl, aryl, heteroaryl, fluorine, chlorine, bromine, hydroxyl, mercapto, cyano, nitro, nitroso, formyl, acyl, COOH, carboxylate, alkylcarbonyloxy, carbamoyl, SO₃H, sulfonate, sulfamino, sulfamide, amidino, NE³E⁴ where E³ and E⁴ are each independently hydrogen, alkyl, cycloalkyl, heterocycloalkyl, aryl or heteroaryl. Halogen substituents are preferably fluorine, chlorine or bromine.

The substituents on the substituted hetaryl groups R¹, R², R³, and R⁴ are preferably selected from alkyl; alkoxy; alkyl or alkoxy whose carbon chain is interrupted by one or more nonadjacent groups selected from -O-, -S-, -NR^{b}-, -C(=O)-, -S(=O)- and/or -S(=O)₂-, wherein R^{b} is hydrogen, alkyl, cycloalkyl, heterocycloalkyl, aryl or heteroaryl; haloalkyl; haloalkoxy; cycloalkyl; fluorine; chlorine; bromine; cyano and nitro.

The substituted hetaryl groups R¹, R², R³, and R⁴ are preferably selected from heteroaryl groups substituted by at least one alkyl group. Alkyl substituted heteroaryl groups may, depending on the size of the aromatic ring system, have one or more (e.g. 1, 2, 3, 4, 5, 6, 7, 8, 9 or more than 9) alkyl substituents. The alkyl substituents on the heteroaryl groups may be unsubstituted or substituted. In this regard, reference is made to the following statements regarding unsubstituted and substituted alkyl. In a preferred embodiment, the heteroaryl groups have exclusively unsubstituted alkyl substituents. The alkyl substituents on the hetaryl groups R¹, R², R³, and R⁴ preferably selected from C₁-C₂₀-alkyl, more preferably C₁-C₁₂-alkyl and most preferably C₁-C₆-alkyl. Examples of alkyl groups are especially methyl, ethyl, n-propyl, isopropyl, n-butyl, isobutyl, sec-butyl, tert-butyl, n-pentyl, neo-pentyl, n-hexyl, n-heptyl, n-octyl, n-nonyl, n-decyl, n-undecyl, n-dodecyl, n-tridecyl, n-tetradecyl, n-hexadecyl, n-octadecyl and n-eicosyl.

R¹, R², R³, and R⁴ are in particular selected from a straight-chain or branched C₁-C₁₂-alkyl group, a cyclohexyl group, a cyclohexyl group, which bear one, two or three C₁-C₆-alkyl groups, a phenyl group, or a phenyl group, which bears one, two or three C₁-C₁₂-alkyl groups.

In a preferred embodiment the present invention is directed to particles of the compound of formula

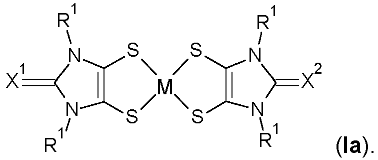

X¹ and X² are each independently of each other sulfur or oxygen; especially X¹ and X² are oxygen, or X¹ is oxygen and X² is sulfur; very especially X¹ and X² are sulfur.

R' is a straight-chain or branched C₁-C₁₂-alkyl group, a cyclohexyl group, a cyclohexyl group, which bear one, two or three C₁-C₆-alkyl groups, a phenyl group, or a phenyl group, which bears one, two or three C₁-C₁₂-alkyl groups.

M is Ni, or Pt, especially Ni.

The particles of the compound of formula (**Ia**) have a D₅₀ in the range of from 40 to 80 nm with D₁₀ being greater than 25 nm, especially a D₅₀ in the range of from 50 to 70 nm with D₁₀ being greater than 30 nm.

In another preferred embodiment the present invention is directed to particles of the compound of formula

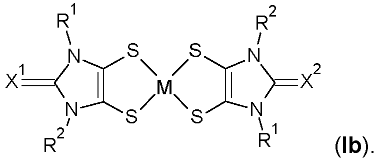

X¹ and X² are each independently of each other sulfur or oxygen; especially X¹ and X² are oxygen, or X¹ is oxygen and X² is sulfur; very especially X¹ and X² are sulfur.

R¹ and R² are a straight-chain or branched C₁-C₁₂-alkyl group, a cyclohexyl group, a cyclohexyl group, which bear one, two or three C₁-C₆-alkyl groups, a phenyl group, or a phenyl group, which bears one, two or three C₁-C₁₂-alkyl groups, with the proviso that R' is different from R². In said embodiment R¹ is preferably a straight-chain or branched C₁-C₁₂-alkyl group, a cyclohexyl group, or a cyclohexyl group, which bear one, two or three C₁-C₆-alkyl groups, and R² is a phenyl group, or a phenyl group, which bears one, two or three C₁-C₁₂-alkyl groups; or R¹ is preferably a straight-chain or branched C₁-C₁₂-alkyl group, and R² is a cyclohexyl group, or a cyclohexyl group, which bear one, two or three C₁-C₆-alkyl groups.

M is Ni, or Pt, especially Ni.

The particles of the compound of formula (Ib) have a D₅₀ in the range of from 40 to 80 nm with D₁₀ being greater than 25 nm, especially a D₅₀ in the range of from 50 to 70 nm with D₁₀ being greater than 30 nm.

Examples of preferred compounds of formula (I) are compounds 1 to 47 listed in claim 7. Among compounds 1 to 45 compounds 1, 8, 39, 41, 46 and 47 are preferred, compounds 1, 8, 41, 46 and 47 are more preferred and compound 1 is most preferred.

Those compounds can be obtained from the corresponding sulfur compounds (X¹, X² = S) by oxidation in a suitable solvent. Suitable oxidation agents are oxygen and oxygen containing gas mixtures, in particular atmospheric oxygen. Suitable solvents are inert under the oxidation conditions. Preferred solvents are halogenated hydrocarbons, e.g. dichloromethane.

Compounds of the general formula (I), wherein X¹ is oxygen and X² is sulfur or oxygen can also be obtained from disubstituted imidazolidine-2,4,5-triones of the formula (II) 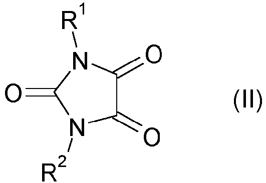 wherein R¹ and R² have one of the aforementioned meanings. Suitable methods are described in WO2008/086931 which is incorporated herein by reference.

The process for the production of the particles according to the present invention comprises
a) providing a mixture of a compound of formula 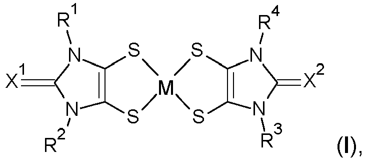
   wherein M is selected from Ni, Pd and Pt,
   X¹ and X² are each independently of each other sulfur or oxygen,
   R¹, R², R³, and R⁴ are independently selected from an unsubstituted, or substituted alkyl group, an unsubstituted or substituted aryl group and an unsubstituted or substituted heteroaryl group, a solvent and an inorganic salt; and
b) kneading the mixture at a temperature of from 20 to 150°C for a sufficient period of time.

In general, the kneading mass contains, per g of the total mass of the compound of formula (I) from 1 to 15 g, preferably from 2 to 8 g of inorganic salt and from 0.3 to 2 g, preferably from 0.5 to 2 g of a solvent.

The solvent is preferably selected from a protic solvent, an aprotic solvent, such as, for example, N,N-dimethylformamide (DMF) and N-Methyl-2-pyrrolidone (NMP); an aliphatic alcohol, a diol, or a polyol, such as, for example, a glycol, or glycerol; and mixtures thereof. Diethylene glycol and triethylene glycol are most preferred.

Suitable salts for salt kneading are water-soluble salts having a solubility of at least 10 g / 100 ml in water. Suitable examples are sodium chloride, potassium chloride, calcium chloride, zinc chloride, aluminum chloride, sodium sulfate, aluminum sulfate and calcium carbonate, with or without water of crystallization. Preferred inorganic salts are sodium chloride and sodium sulfate, more preferred is sodium chloride. Typically, technical-grade salts with or without preceding micronization are used. The salts preferably have an average particle size of from 5 to 200 µm, more preferably from 10 to 50 µm.

The kneading temperature is generally of from 20 to 150°C, preferably 30 to 110°C.

The salt kneading step should be carried out for a sufficient period of time to allow the particles to attain optimum stability, pigmentary size and distribution. The period of time is not critical and may range from 2 to 15 hours, preferably 2 to 10 hours, in particular from 2 to 6 hours.

The speed or rotation rate is appropriately selected in such a way that the kneading mass is moved homogeneously and with uniform shear. The product resulting after kneading may be stirred and granulated in water to remove salt and organic liquid and isolated by common methods, like filtering, washing usually salt free with water and drying, preferably at a temperature of from 50 to 90°C.

Any kneader for salt kneading known in the art may be used, for example, common double-shaft kneaders, such as Z-blade kneaders, planetary kneaders or screw kneaders, but also single-shaft kneaders, high speed mixers or extruders are likewise possible.

The particles of the compounds of formula (I), especially of formula (la) and (Ib), have a number average particle size in the range of from 10 nm to 80 nm, preferably from 20 nm to 70 nm, more preferably 30 to 60 nm with standard deviation being less than 50 nm, especially less than 30 nm, very especially less than 25 nm. The particle size is measured with transmission electron microscopy (TEM).

The particles of the compounds of formula (I), especially of formula (la), (Ib) and (Ic), have a number average particle size in the range of from 10 nm to 60 nm, preferably from 10 nm to 50 nm, more preferably 20 to 40 nm with standard deviation being less than 50%, especially less than 30 %, very especially less than 20 %. The particle size is measured with transmission electron microscopy (TEM).

TEM analysis of dispersions was performed on "Libra 120", an instrument from ZEISS in bright field mode at an electron beam acceleration voltage of 120 kV. The TEM was used with an energy filter for better contrast. At least 2 representative images with scale in the same magnification were recorded in order to characterize the dominate particle morphology for each sample. The minimal feret diameter of the particles was determined with the software "ImageJ", which is based on the measurement of at least 4800 randomly selected particles.

The particles of the compounds of formula (I) according to the invention are suitable for welding transparent at least translucent plastic materials. In laser welding, plastics components are welded to one another. The plastics components to be fused may have any shape. For example, at least one of the plastics components may be a film. The laser welding is preferably carried out using a YAG laser or using a diode laser emitting within the absorption range of the afore-mentioned IR absorber of the formula (I). The concentration of the IR absorber of the formula (I) or of IR absorber mixtures is e.g. from 5 to 500 ppm, preferably from 10 to 200 ppm. The employed plastic materials may be colourless or coloured. In principle, the plastic components to be fused may be composed of the same polymer or of different polymers. Preferably, the plastic components employed for laser welding are selected from thermoplastic polymers. However, it is also possible that neither of the plastic components to be fused is composed of thermoplastic; however, a coating of at least one part with a thermoplastic comprising the particles of the compound of the formula (I) is required.

The plastic components employed for laser welding preferably comprise or consist of at least one polymer selected from polyolefins, polyolefin copolymers, polytetrafluoroethylenes, ethylene-tetrafluoroethylene copolymers, polyvinyl chlorides, polyvinylidene chlorides, polyvinyl alcohols, polyvinyl esters, polyvinyl alkanals, polyvinyl ketals, polyamides, polyimides, polycarbonates, polycarbonate blends, polyesters, polyester blends, poly(meth)acrylates, poly(meth)acrylate-styrene copolymer blends, poly(meth)acrylate-polyvinylidene difluoride blends, polyurethanes, polystyrenes, styrene copolymers, polyethers, polyether ketones and polysulfones and mixtures thereof.

Preference is given to matrix polymers from the group of the polyolefins, polyolefin copolymers, polyvinyl alkanals, polyamides, polycarbonates, polycarbonate-polyester blends, polycarbonate-styrene copolymer blends, polyesters, polyester blends, poly(meth)acrylates, poly(meth)acrylate-styrene copolymer blends, poly(meth)acrylate-polyvinylidene difluoride blends, styrene copolymers and polysulfones and mixtures thereof.

Particularly preferred polymers are transparent or at least translucent. Examples include: polypropylene, polyvinylbutyral, nylon-[6], nylon-[6,6], polycarbonate, polycarbonate-polyethylene terephthalate blends, polycarbonate-polybutylene terephthalate blends, polycarbonate-acrylonitrile/styrene/acrylonitrile copolymer blends, polycarbonate-acrylonitrile/butadiene/styrene copolymer blends, polymethyl methacrylate-acrylonitrile/butadiene/styrene copolymer blends (MABS), polyethylene terephthalate, polybutylene terephthalate, polymethyl methacrylate, impact-modified polymethyl methacrylate, polybutyl acrylate, polymethyl methacrylate-polyvinylidene difluoride blends, acrylonitrile/butadiene/styrene copolymers (ABS), styrene/acrylonitrile copolymers (SAN), polyphenylenesulfone and mixtures comprising 2 or more (e.g. 2, 3, 4, 5) of the afore-mentioned polymers.

Suitable polymer preparations for laser welding comprise
A) a thermoplastic matrix polymer suitable for forming the plastics parts,
B) particles of the compound of the formula (I) as defined before,
C) optionally at least one further additive.

Those polymer preparations for laser welding are likewise in accordance with the invention and are suitable for producing fusion-bonded plastic parts with the aid of laser radiation whose wavelength is outside the visible region.

Polymer preparations for laser welding may advantageously be produced by a conventional extrusion or kneading process. The components B), and, if present, C) may be mixed from the outset, in the weight ratio corresponding to the desired end concentration, with the matrix polymer A) (direct compounding), or a distinctly higher concentration of B) and, if present, C) may initially be selected and the concentrate formed (masterbatch) subsequently diluted with further matrix polymer A) in the course of the manufacture of the parts to be fused.

Suitable additives C) are UV stabilizers, antioxidants, processing plasticizers, etc.

In addition, the polymer preparations for laser welding may comprise at least one colorant for establishing a desired hue as additive, especially transparent organic pigments and in particular dyes, for example C.I. Pigment Yellow 93, 110, 128, 138, 139, 147, 155, 180, 183, 185 192 196 and 215, C.I. Pigment Orange 61, 64, 70, 71, 79, C.I. Pigment Red 122, 144, 149, 178 and 179, 181, 202, 214, 220, 263, C.I. Pigment Violet 19, 23, 29 and 37 , C.I. Pigment Blue 15, 15:1, 15:3 and 15:4, 60, C.I. Pigment Green 7 and 36, C.I. Pigment Black, C.I. Solvent Yellow 14, 21, 93, 130, 133, 145, 163, C.I. Solvent Red 52, 135, 195, 213, 214 and 225, C.I. Solvent Blue 35, 45, 67, 68, 97, 104, 122, 132, C.I. Solvent Violet 13, 46, 49, C.I. Solvent Green 3, 5 and 28, C.I. Solvent Orange 47, 60, 86, 114, and 163, C.I. Solvent Brown 35, 53, and also C.I. Disperse Yellow 54, 87, 201, C.I. Disperse Orange 30, C.I. Disperse Red 60 and C.I. Disperse Violet 57.

A further possible additive group is that of additives which likewise modify the visual appearance, the mechanical properties or else the tactile properties, for example matting agents, such as titanium dioxide, chalk, barium sulfate, zinc sulfide, fillers, such as nanoparticulate silicon dioxide, aluminium hydroxide, clay and other sheet silicates, glass fibers and glass spheres.

An especially suitable field of application is the use of the compound of formula (I) in security printing.

The particles of the compound of the general formula (I) has at least one of the following advantageous properties:
- good fastness to chemicals, in particular fastness to bleaching with hypochlorite and fastness to solvents (like toluene, acetone or dichloromethane),
- good fastness to boiling water,
- good fastness to light,
- almost colourless (i.e. minimal absorption in the VIS range of the spectrum (from 400 to 700 nm))
- good heat stability,
- high absorbing properties,
- high compatibility with a multiplicity of formulations, in particular printing ink formulations used especially in security printing.

The particles of the compound of general formula (I) can be used inter alia for security printing, invisible and/or IR readable bar codes, the laser-welding of plastics, the curing of surface-coatings using IR radiators, the drying and curing of print, the fixing of toners on paper or plastics, optical filters for plasma display panels, laser marking of paper or plastics, the heating of plastic preforms, 3D printing and for heat shielding applications.

Some examples of three-dimensional (3D) printing may utilize a fusing agent (including an energy absorber) to pattern polymeric build material. The fusing agent is capable of absorbing radiation and converting the absorbed radiation to thermal energy, which in turn coalesces/fuses the polymeric build material that is in contact with the fusing agent.

Accordingly, the present invention is directed to fusing agents, comprising particles of a compound of formula (I), especially a compound of formula (la) and (Ib). The composition of the fusing agents is, for example, described in WO2020005200, WO2019245589, WO2019245518, WO2019245517, WO2019245535, WO2019245534, WO2019245516 and US2019382429.

In addition, the present invention is directed to a consumable material for use in an additive manufacturing system, the consumable material comprising:
at least one polymer comprising:
particles of at least one compound of formula (I), especially at least one compound of formula (la) and/or (Ib).

In addition, the present invention is directed to a consumable assembly for use in an extrusion-based additive manufacturing system, the consumable assembly comprising:
a container portion;
a consumable filament at least partially retained by the container portion, the consumable filament comprising:
   at least one polymer,
   particles of at least one compound of formula (I), especially at least one compound of formula (la) and/or (Ib).

The consumable filament may have a core comprising the at least one polymer and a coating comprising particles of at least one compound of formula (I) (WO2015130401).

The at least one polymer may be a meltable polymer which is selected from the group consisting of polyurethane, polyester, polyalkylene oxide, plasticized PVC, polyamide, protein, PEEK, PEAK, polypropylene, polyethylene, thermoplastic elastomer, POM, polyacrylate, polycarbonate, polymethylmethacrylate, polystyrene or a combination of at least two of these.

A process for producing an article by means of an additive manufacturing method from the consumable material comprises at least temporarily exposing the consumable material to infrared radiation in the wavelength range between 600 nm and 1700 nm.

The present invention is also directed to an article obtainable by the process.

In a further aspect, the invention provides a printing ink formulation for security printing, comprising the particles of the compound of the formula (I) as defined above.

In a specific embodiment the printing ink formulation, for security printing, comprises
a) the particles of the compound of the formula (I) as defined above,
b) a polymeric binder,
c) optionally at least one solvent,
d) optionally at least one colorant, and
e) optionally at least one further additive.

More specific the printing ink formulation comprises
a) 0.0001 to 25 % by weight of particles of at least one compound of the formula (I) as defined above,
b) 5 to 74 % by weight of at least one polymeric binder,
c) 0 to 94.9999 % by weight of at least one solvent,
d) 0 to 25 % by weight of at least one colorant, and
e) 0 to 25 % by weight of at least one further additive,
wherein the sum of components a) to e) adds up to 100%.

Also an aspect of the invention is a process for the manufacture of a security document comprising the steps of printing on a substrate a printing ink formulation as described above.

In another aspect, the invention provides a security document, comprising a substrate and the particles of the compound of the formula (I) as defined above. The security document may be a bank note, a passport, a check, a voucher, an ID- or transaction card, a stamp and a tax label.

Yet in a further aspect, the invention provides a security document, obtainable by a printing process, wherein a printing ink formulation is employed that comprises the particles of the compound of the formula (I) as defined above.

The particles of the IR absorber of formula (I) can also be used in the form of a mixture, comprising at least one compound of the general formula (I) and at least one further IR absorber different from compounds of the general formula (I). Suitable further IR absorbers are in principle all known classes of IR absorbers that are compatible with the compounds of the general formula (I). Preferred further IR absorbers are selected from polymethines, phthalocyanines, quinone-diimmonium salts, aminium salts, rylenes, inorganic IR absorbers and mixtures thereof. Further polymethine IR absorbers are preferably selected from cyanines, squaraines, croconaines and mixtures thereof. Further inorganic IR absorbers are preferably selected from indium tin oxide, antimony tin oxide, lanthanum hexaboride, tungsten bronzes, copper salts etc.

The IR absorbers can be generally used in a concentration of from 10 ppm to 25%, preferably 100 ppm to 10%, depending on the chosen application.

The afore-mentioned IR absorbers of the formula (I) and IR absorber mixtures are especially suitable for security printing.

Security printing is the field that deals with the printing of items such as currency, passports, tamper-evident labels, stock certificates, postage stamps, identity cards, etc. The main goal of security printing is to prevent forgery, tampering or counterfeiting.

In the field of automated banknote processing, IR-absorption plays an important role. Most of the actually circulating currency carries not only visibly coloured printings, but also specific features which are only detectable in the infrared part of the spectrum. Generally, these IR-features are implemented for use by automatic currency processing equipment, in banking and vending applications (automatic teller machines, automatic vending machines, etc.), in order to recognize a determined currency bill and to verify its authenticity, in particular to discriminate it from replicas made by colour copiers.

All security documents are required to have good stability and durability. In the case of bank notes, these requirements are extreme, as bank notes are subjected to toughest use conditions by the public - they are subjected to material stress by folding, crumpling etc., subjected to abrasion, exposed to weather, exposed to bodily fluids such as perspiration, laundered, dry-cleaned, ironed etc. - and, after having been subjected to this, are expected to be as legible as when they started. Furthermore, it is essential that the documents nevertheless should have a reasonable life time, ideally of some years, despite suffering the afore-mentioned conditions. During this time, the documents, and thus the inks on them (including invisible security markings), should be resistant to fading or colour change. Hence, any ink used in a security printing process should, when cured, be robust, water-resistant, resistant to various chemicals and flexible. Moreover, as certain states are moving away from the use of paper as the substrate for bank notes, the employed printing ink formulations should be useable on plastics as well as paper.

In one aspect the present invention is directed to the use of particles of the compound of the formula (I) for security printing, especially security printing of bank notes. The particles of the compound of formula (I) may exhibit improved resistance against chemicals and solvents as well as high light stability, particularly against UV light.

Advantageously, the particles of the compound of the formula (I) may be used in a printing ink formulation for security printing to improve the fastness properties of the obtained print, in particular to improve the fastness to UV-light, chemicals, solvents and/or boiling water, without sacrificing the desired IR absorption properties.

In security printing, the particles of the compound of formula (I) is added to a printing ink formulation. Suitable printing inks are water-based, oil-based, solvent-based, UV-curable and/or electron beam curable printing inks, based on pigment or dye, for inkjet printing, flexographic printing, screen printing, intaglio printing, offset printing, laser printing or letterpress printing and for use in electrophotography. Printing inks for these printing processes usually comprise solvents, binders, and also various additives, such as plasticizers, antistatic agents or waxes. Printing inks for offset printing, letterpress printing and intaglio printing are usually formulated as high-viscosity paste printing inks, whereas printing inks for flexographic printing, gravure and ink jet printing are usually formulated as liquid printing inks with comparatively low viscosity.

In the context of the present invention, the expression "printing ink" also encompasses formulations that in addition to particles of at least one IR absorber of the general formula (I) comprise a colorant. The expression "printing ink" also encompasses printing lacquers that comprise no colorant.

The printed layer comprising at least one IR absorber can be on, under or in between further printed and/or laminated layers. These further layers can be functional layers e.g. magnetic, luminescent, conductive, NIR transparent, colored or metallic layers, adhesives, overprint varnishes, or release layers. These layers can also be structured or patterned. These further layers can also have optical variable effects, e.g. angle dependant colorshifts, they can contain diffractive effects, like holograms, lenses, plas-monic effects, dynamic effects caused e.g. by micromirrors, microprisms, lenticular and/or micolenses.

The printed layer comprising at least one IR absorber can be applied on the banknote substrate itself or be part of a security thread, a banknote window or onto the banknote laminated foil elements.

Suitable components of printing inks are conventional and are well known to those skilled in the art. Examples of such components are described in "Printing Ink Manual", fourth edition, Leach R. H. et al. (eds.), Van Nostrand Reinhold, Wokingham, (1988). Details of printing inks and their formulation are also disclosed in "Printing Inks"-Ullmann's Encyclopedia of Industrial Chemistry, Sixth Edition, 1999 Electronic Release. A formulation of an IR-absorbing intaglio ink formulation is described in US 20080241492 A1. The disclosure of the afore-mentioned documents is incorporated herein by reference.

The printing ink formulation according to the invention contains in general from 0.0001 to 25% by weight, preferably from 0.001 to 15% by weight, in particular from 0.1 to 10% by weight, based on the total weight of the printing ink formulation, of the particles of the compound of formula (I), component a).

The particles of the compound of formula (I) are present in the printing ink formulation in dissolved form or in solid form (in a finely divided state). Due to their pigment properties te solid form is preferred.

The printing ink formulation according to the invention contains in general from 5 to 74% by weight, preferably from 10 to 60% by weight, more preferably from 10 to 30% by weight, based on the total weight of the printing ink formulation, of component b).

Suitable polymeric binders b) for the printing ink formulation according to the invention are for example selected from natural resins, phenol resin, phenol-modified resins, alkyd resins, polystyrene homo- and copolymers, terpene resins, silicone resins, polyurethane resins, urea-formaldehyde resins, melamine resins, polyamide resins, polyacrylates, polymethacrylates, chlorinated rubber, vinyl ester resins, acrylic resins, epoxy resins, nitrocellulose, hydrocarbon resins, cellulose acetate, and mixtures thereof.

The printing ink formulation according to the invention can also comprise components that form a polymeric binder by a curing process. Thus, the printing ink formulation according to the invention can also be formulated to be energy-curable, e.g. able to be cured by UV light or EB (electron beam) radiation. In this embodiment, the binder comprises one or more curable monomers and/oligomers. Corresponding formulations are known in the art and can be found in standard textbooks such as the series "Chemistry & Technology of UV & EB Formulation for Coatings, Inks & Paints", published in 7 volumes in 1997-1998 by John Wiley & Sons in association with SITA Technology Limited.

Suitable monomers and oligomers (also referred to as prepolymers) include epoxy acrylates, acrylated oils, urethane acrylates, polyester acrylates, silicone acrylates, acrylated amines, and acrylic saturated resins. Further details and examples are given in "Chemistry & Technology of UV & EB Formulation for Coatings, Inks & Paints", Volume II: Prepolymers & Reactive Diluents, edited by G Webster.

If a curable polymeric binder is employed, it may contain reactive diluents, i.e. monomers which act as a solvent and which upon curing are incorporated into the polymeric binder. Reactive monomers are typically chosen from acrylates or methacrylates, and can be monofunctional or multifunctional. Examples of multifunctional monomers include polyester acrylates or methacrylates, polyol acrylates or methacrylates, and polyether acrylates or methacrylates.

In the case of printing ink formulations to be cured by UV radiation, it is usually necessary to include at least one photoinitiator to initiate the curing reaction of the monomers upon exposure to UV radiation. Examples of useful photoinitiators can be found in standard textbooks such as "Chemistry & Technology of UV & EB Formulation for Coatings, Inks & Paints", Volume III, "Photoinitiators for Free Radical Cationic and Anionic Polymerisation", 2nd edition, by J. V. Crivello & K. Dietliker, edited by G. Bradley and published in 1998 by John Wiley & Sons in association with SITA Technology Limited. It may also be advantageous to include a sensitizer in conjunction with the photoinitiator in order to achieve efficient curing.

The printing ink formulation according to the invention contains in general from 1 to 94.9999 % by weight, preferably from 5 to 90 % by weight, in particular from 10 to 85% by weight, based on the total weight of the printing ink formulation, of a solvent c).

Suitable solvents are selected from water, organic solvents and mixtures thereof. For the purpose of the invention, reactive monomers which also act as solvents are regarded as part of the afore-mentioned binder component b).

Examples of solvents comprise water; alcohols, e.g. ethanol, 1-propanol, 2-propanol, ethylene glycol, propylene glycol, diethylene glycol and ethoxy propanol; esters, e.g. ethyl acetate, isopropyl acetate, n-propyl acetate and n-butyl acetate; hydrocarbons, e.g. toluene, xylene, mineral oils and vegetable oils, and mixtures thereof.

The printing ink formulation according to the invention may contain an additional colorant d). Preferably, the printing ink formulation contains from 0 to 25% by weight, more preferably from 0.1 to 20% by weight, in particular from 1 to 15% by weight, based on the total weight of the printing ink formulation, of a colorant d).

Suitable colorants d) are selected conventional dyes and in particular conventional pigments. The term "pigment" is used in the context of this invention comprehensively to identify all pigments and fillers, examples being colour pigments, white pigments, and inorganic fillers. These include inorganic white pigments, such as titanium dioxide, preferably in the rutile form, barium sulfate, zinc oxide, zinc sulfide, basic lead carbonate, antimony trioxide, lithopones (zinc sulfide + barium sulfate), or coloured pigments, examples being iron oxides, carbon black, graphite, zinc yellow, zinc green, ultramarine, manganese black, antimony black, manganese violet, Paris blue or Schweinfurt green. Besides the inorganic pigments the printing ink formulation of the invention may also comprise organic colour pigments, examples being sepia, gamboge, Cassel brown, toluidine red, para red, Hansa yellow, indigo, azo dyes, anthraquinonoid and indigoid dyes, and also dioxazine, quinacridone, phthalocyanine, isoindolinone, and metal complex pigments. Also suitable are synthetic white pigments with air inclusions to increase the light scattering, such as the Rhopaque^{®} dispersions. Suitable fillers are, for example, aluminosilicates, such as feldspars, silicates, such as kaolin, talc, mica, magnesite, alkaline earth metal carbonates, such as calcium carbonate, in the form for example of calcite or chalk, magnesium carbonate, dolomite, alkaline earth metal sulfates, such as calcium sulfate, silicon dioxide, etc.

The printing ink formulation according to the invention may contain at least one additive e). Preferably, the printing ink formulation contains from 0 to 25% by weight, more preferably from 0.1 to 20% by weight, in particular from 1 to 15% by weight, based on the total weight of the printing ink formulation, of at least one component e).

Suitable additives (component e)) are selected from plasticizers, waxes, siccatives, antistatic agents, chelators, antioxidants, stabilizers, adhesion promoters, surfactants, flow control agents, defoamers, biocides, thickeners, etc. and combinations thereof. These additives serve in particular for fine adjustment of the application-related properties of the printing ink, examples being adhesion, abrasion resistance, drying rate, or slip.

The printing ink formulations according to the invention are advantageously prepared in a conventional manner, for example by mixing the individual components. As mentioned earlier, the particles of the compound of formula (I) is present in the printing ink formulations in a dissolved or finely divided solid form. Additional colorants may be employed in the printing ink formulation of the invention or in a separate ink formulation. When additional colorants are to be employed in a separate formulation, the time of application of the printing ink formulation according to the invention is usually immaterial. The printing ink formulation according to the invention can for example be applied first and then be overprinted with conventional printing inks. But it is also possible to reverse this sequence or, alternatively, to apply the printing ink formulation according to the invention in a mixture with conventional printing inks. In every case the prints are readable with suitable light sources.

Primers can be applied prior to the printing ink formulation according to the invention. By way of example, the primers are applied in order to improve adhesion to the substrate. It is also possible to apply additional printing lacquers, e.g. in the form of a covering to protect the printed image. Additional printing lacquers may also be applied to serve aesthetic purposes, or serve to control application-related properties. By way of example, suitably formulated additional printing lacquers can be used to influence the roughness of the surface of the substrate, the electrical properties, or the water-vapour-condensation properties. Printing lacquers are usually applied in-line by means of a lacquering system on the printing machine employed for printing the printing ink formulation according to the invention.

The printing ink formulations according to the invention are also suitable for use in multilayer materials. Multilayer materials are e.g. composed of two or more plastics foils, such as polyolefin foils, metal foils, or metallised plastics foils, which are bonded to one another, by way of example, via lamination or with the aid of suitable laminating adhesives. These composites may also comprise other functional layers, such as odour-barrier layers or water-vapour barriers.

The printing ink formulations may additionally comprise one or more UV absorbers. UV absorbers are well known in the plastics, coatings and cosmetic industry. Examples for suitable UV absorbers are subsequently given.

2-(2'-Hydroxyphenyl)benzotriazoles, for example 2-(2'-hydroxy-5'-methylphenyl)-benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chloro-benzotriazole, 2-(3'-tert-butyl-2'-hydroxy-5'-methylphenyl)-5-chloro-benzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis-(α,α-dimethylbenzyl)-2'-hydroxyphenyl)benzotriazole, 2-(3'-tert-butyl-2'-hydroxy-5'-(2-octyloxycarbonylethyl)phenyl)-5-chloro-benzotriazole, 2-(3'-tert-butyl-5'-[2-(2-ethylhexyloxy)-carbonylethyl]-2'-hydroxyphenyl)-5-chloro-benzotriazole, 2-(3'-tert-butyl-2'-hydroxy-5'-(2-methoxycarbonylethyl)phenyl)-5-chloro-benzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenylbenzotriazole, 2,2'-methylene-bis[4-(1,1,3,3-tetramethylbutyl)-6-benzotriazole-2-ylphenol]; the transesterification product of 2-[3'-tert-butyl-5'-(2-methoxycarbonylethyl)-2'-hydroxyphenyl]-2H-benzotriazole with polyethylene glycol 300; 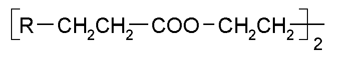 , where R = 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)-phenyl]benzotriazole; 2-[2'-hydroxy-3'-(1,1,3,3-tetramethylbutyl)-5'-(α,α-dimethylbenzyl)-phenyl]benzotriazole.

2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.

Esters of substituted and unsubstituted benzoic acids, for example 4-tert-butyl-phenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.

Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxy-cinnamate, methyl □-carbomethoxy-p-methoxycinnamate, N-(β-carbomethoxy-β-cyanovinyl)-2-methylindoline, neopentyl tetra(α-cyano-β,β-diphenylacrylate.

Oxamides, for example 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxy-disubstituted oxanilides and mixtures of o- and p-ethoxy-disubstituted oxanilides.

2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxy-phenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypro-poxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxy-propoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxypropyloxy]phenyl}-4,6-bis(2,4-dimethylphenyl)-1,3,5-triazine, 2,4-bis(4-[2-ethylhexyloxy]-2-hydroxyphenyl)-6-(4-methoxyphenyl)-1,3,5-triazine.

Various features and aspects of the present invention are illustrated further in the examples that follow. While these examples are presented to show one skilled in the art how to operate within the scope of this invention, they are not to serve as a limitation on the scope of the invention where such scope is only defined in the claims. Unless otherwise indicated in the following examples and elsewhere in the specification and claims, all parts and percentages are by weight, temperatures are in degrees centigrade and pressures are at or near atmospheric.

### Examples

### Example 1

### a) Bis(diphenylimidazolidinetrithione-κS4, κS5)-, (SP-4-1)-nickel(II) (cpd. CC-1 (pigment))

### Preparation of compound (CC-1) (see Example 1 of WO2012/069518):

The compound is known from Eur. J. Inorg. Chem. 2003, 1939 - 1947 and its preparation is described therein.

1,3-Diphenyl-4,5-dioxo-imidazoline is reacted under reflux with metallic nickel and Lawesson's reagent in toluene.

Using chlorobenzene instead of toluene leads to a higher yield. Absorption maximum (chloroform): 1023 nm

The obtained particles of compound (CC-1 (pigment)) have a D₅₀ of 25.4 nm with D₁₀ being 16 nm. The number average particle size is 30.2 nm with standard deviation being 18.5 nm. The particle size is measured with transmission electron microscopy (TEM).

### b) Bis(diphenylimidazolidinetrithione-κS4, κS5)-, (SP-4-1)-nickel(II) (cpd. 1 (pigment kneaded))

70 g of compound CC-1, 290 g sodium chloride and 80 g diethylene glycol are added subsequently at room temperature into a kneader and thoroughly mixed.

Kneading is carried out for 6 hours under ambient conditions.

Then, at 20°C, 500 mL of deionized water is added in portions and mixing is continued for three more hours. Afterwards, the mixture is filtrated, washed with 5 L of deionized water and dried in an oven. Yield: 58 g brownish-black powder.

The obtained particles of compound (1) have a D₅₀ of 60.4 nm with D₁₀ being 38 nm. The number average particle size is 65.5 nm with standard deviation being 25.2 nm. The particle size is measured with transmission electron microscopy (TEM).

TEM analysis of dispersions was performed on "Libra 120", an instrument from ZEISS in bright field mode at an electron beam acceleration voltage of 120 kV. The TEM was used with an energy filter for better contrast. At least 2 representative images with scale in the same magnification were recorded in order to characterize the dominate particle morphology for each sample. The minimal feret diameter of the particles was determined with the software "ImageJ", which is based on the measurement of at least 4800 randomly selected particles.

### Offset printing procedure

An offset ink is prepared by mixing the following components by means of an automatic pigment muller:

| | |
|---|---|
| Offset varnish | 1000 mg |
| IR absorber (cpd. **CC-1** (pigment), or cpd. **1** (pigment kneaded)) | 40 mg |
| Siccative | 20 mg |

Immediately afterwards the freshly prepared offset ink is printed onto uncoated paper with a printability tester (IGT Orange Proofer).

The remission then is measured with the help of a spectrophotometer. Reference is made to Table 1.

**Table 1**

| **Lambda** | **Abs [%]** | **Abs [%]** | **Lambda** | **Abs [%]** | **Abs [%]** |
|---|---|---|---|---|---|
| | **Cpd. CC-1** | **Cpd. 1** | | **Cpd. CC-1** | **Cpd. 1** |
| **1100 nm** | **25%** | **42%** | **940 nm** | **44%** | **91%** |
| 1090 nm | 26% | 43% | 930 nm | 45% | 95% |
| 1080 nm | 26% | 43% | 920 nm | 46% | 98% |
| 1070 nm | 26% | 44% | 910 nm | 47% | 99% |
| 1060 nm | 27% | 45% | 900 nm | 47% | 100%¹⁾ |
| 1050 nm | 27% | 46% | 890 nm | 45% | 99% |
| 1040 nm | 28% | 48% | 880 nm | 44% | 97% |
| 1030 nm | 29% | 51% | 870 nm | 41% | 94% |
| 1020 nm | 30% | 54% | 860 nm | 38% | 88% |
| 1010 nm | 31% | 57% | 850 nm | 35% | 83% |
| 1000 nm | 32% | 61% | 840 nm | 31% | 76% |
| 990 nm | 34% | 65% | 830 nm | 27% | 68% |
| 980 nm | 36% | 70% | 820 nm | 25% | 62% |
| 970 nm | 37% | 75% | 810 nm | 23% | 56% |
| 960 nm | 40% | 81% | 800 nm | 21% | 52% |
| 950 nm | 42% | 86% | | | |

| | | | | | |
|---|---|---|---|---|---|
| ¹⁾ Absorption (= 100 - % remission) of compound 1 at 900 nm is set to 100 %. The other absorption values are given in relation to absorption of compound 1 at 900 nm. | | | | | |

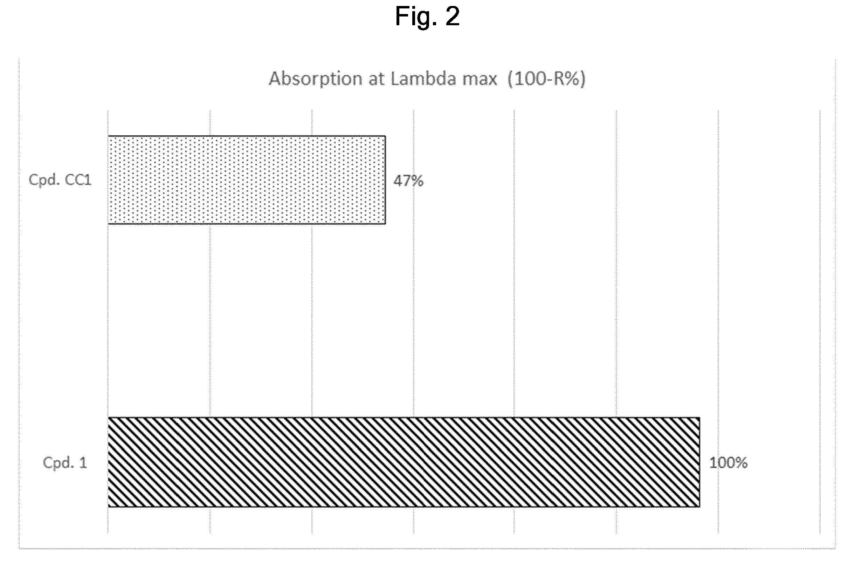

As evident from Table 1 and Fig. 2 compound 1 has a higher IR absorbance in comparison to compound CC-1.

## Claims

1. Particles of a compound of formula 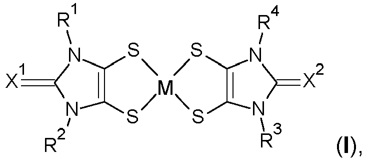 wherein
M is selected from Ni, Pd and Pt,
X¹ and X² are each independently of each other sulfur or oxygen,
R¹, R², R³, and R⁴ are independently selected from an unsubstituted, or substituted alkyl group, an unsubstituted, or substituted cycloalkyl group, an unsubstituted or substituted aryl group and an unsubstituted or substituted heteroaryl group,
wherein the particles have a median particle size (D₅₀) in the range of from 30 nm to 90 nm-with D10 being greater than 20 nm.

2. The particles according to claim 1, wherein the compound of formula (I) is a compound of formula 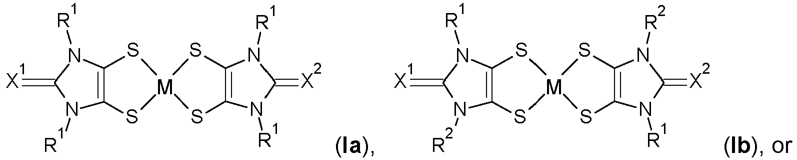 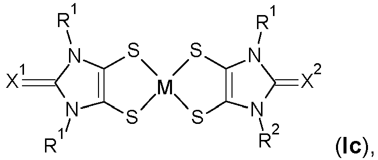 wherein M, X¹, X², R¹ and R² are defined in claim 1.

3. The particles according to claim 2, wherein in the compound of formula (Ia) R' is an unsubstituted, or substituted alkyl group, an unsubstituted or substituted aryl group.

4. The particles according to claim 2, wherein in the compound of formula (Ib), or (Ic) R' is an unsubstituted, or substituted alkyl group and R² an unsubstituted or substituted aryl group.

5. The particles according to any of claims 1 to 4, wherein X¹ and X² are sulfur.

6. The particles according to any of claims 1 to 5, wherein M is Ni.

7. The particles according to any of claims 1 to 6, wherein the compound of formula (I) is a compound of formula

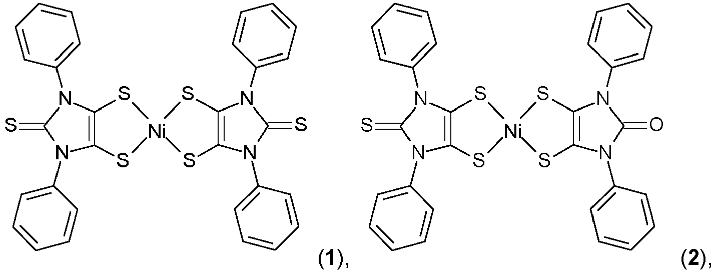

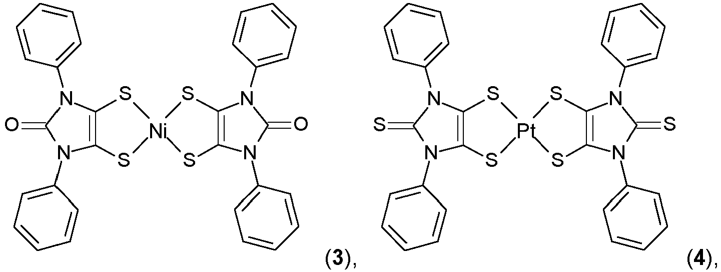

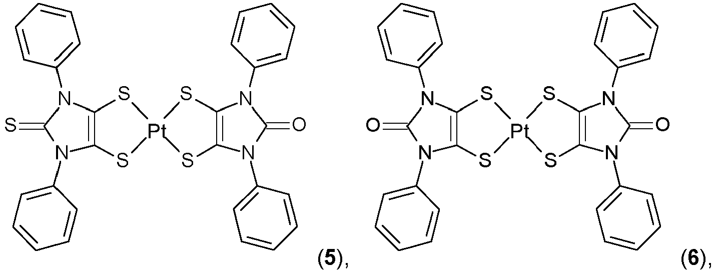

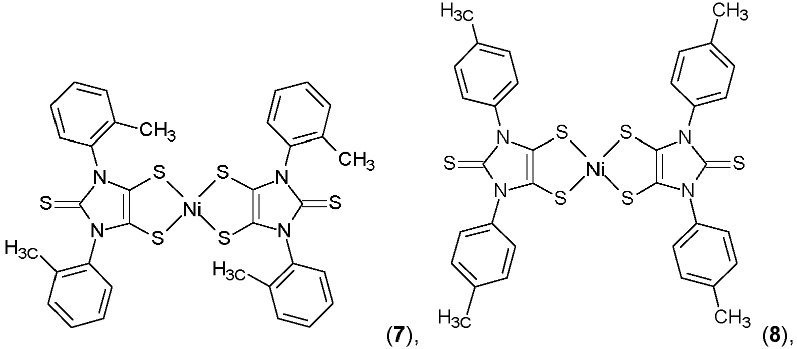

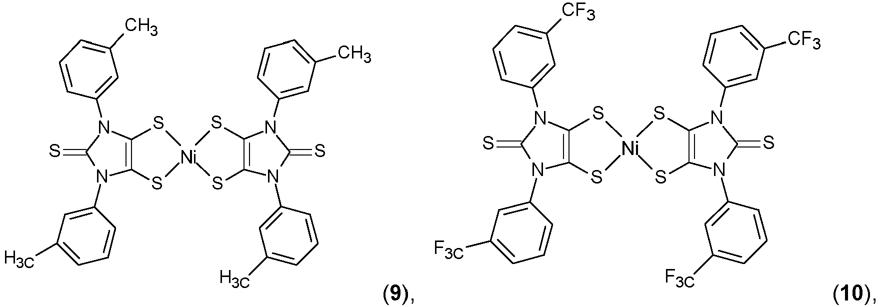

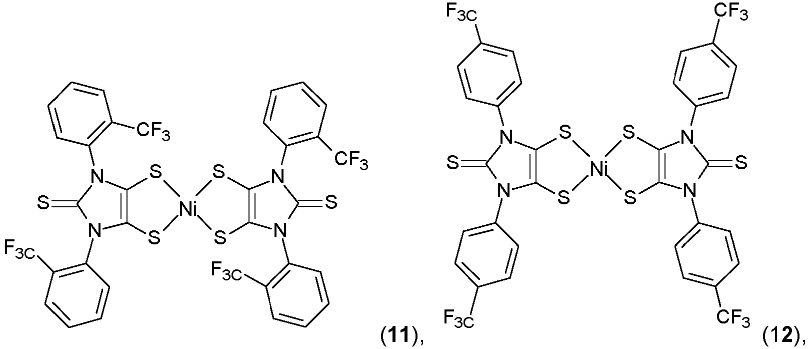

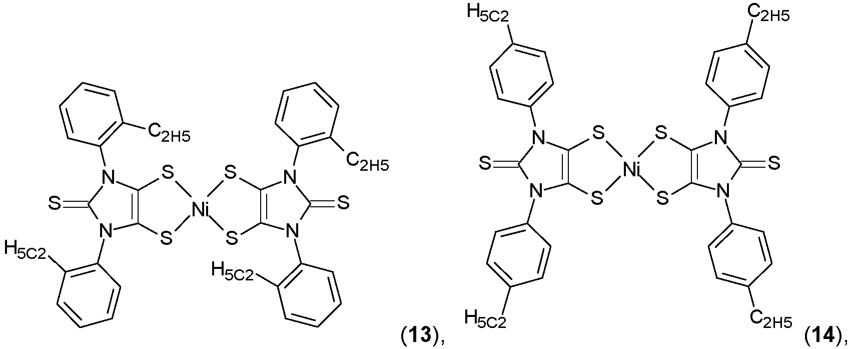

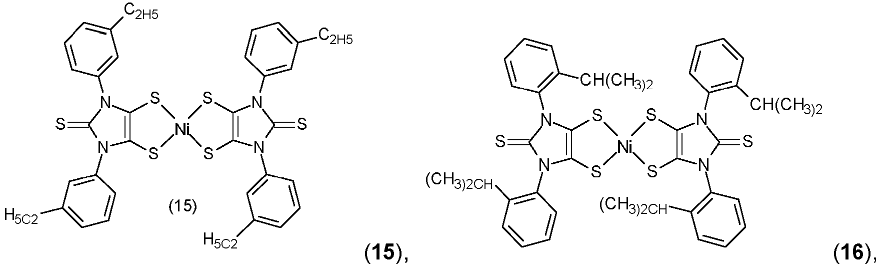

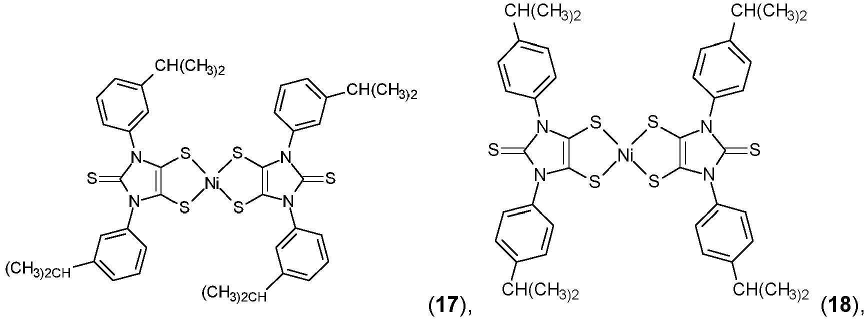

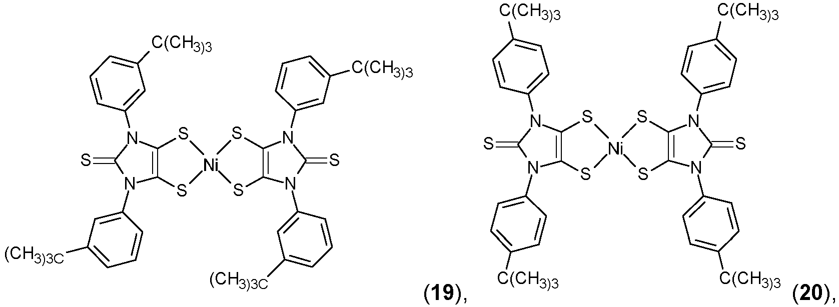

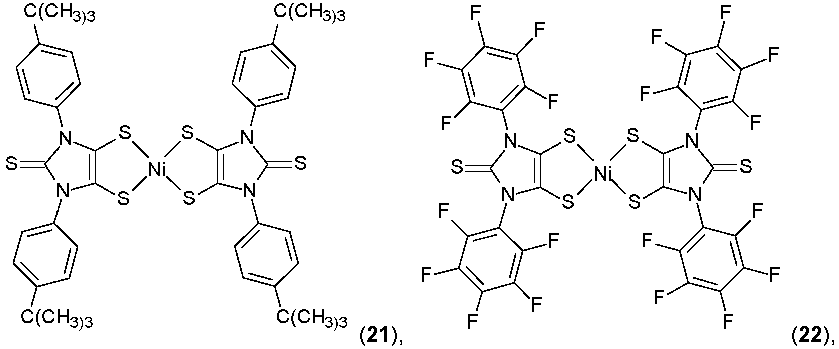

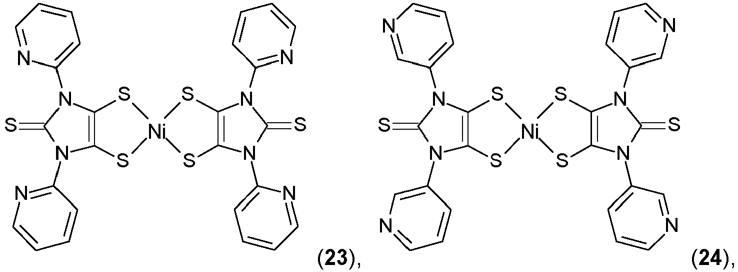

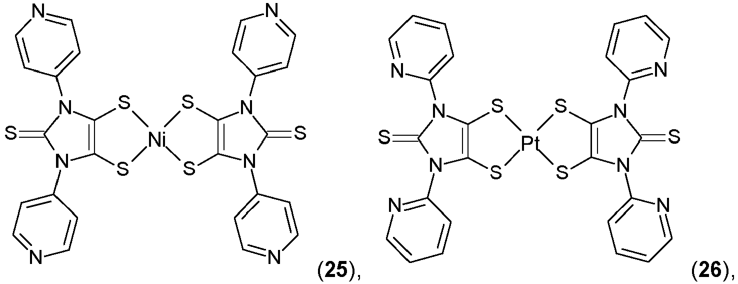

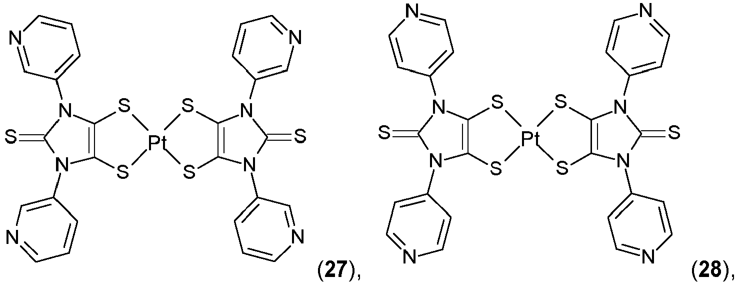

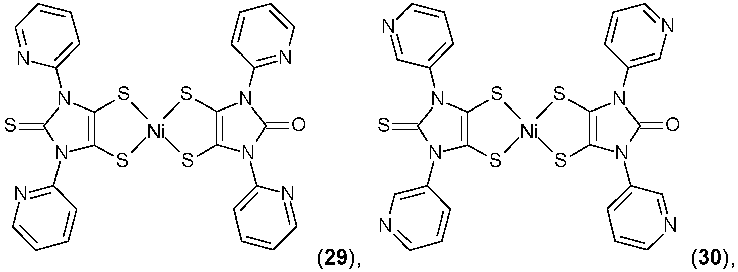

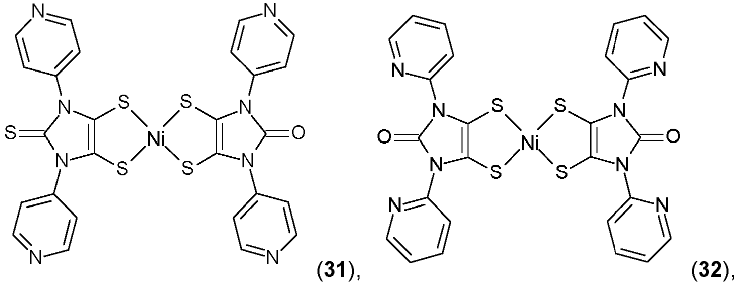

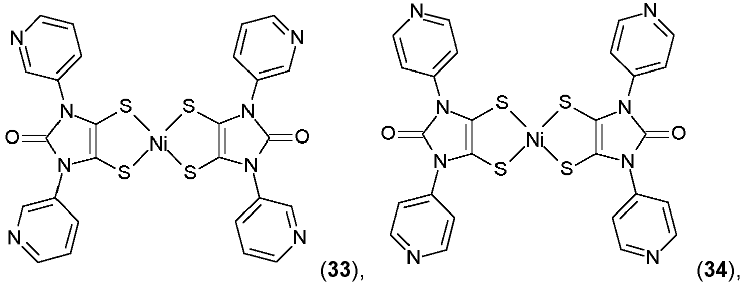

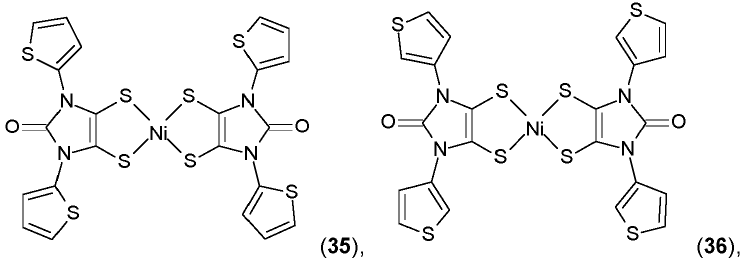

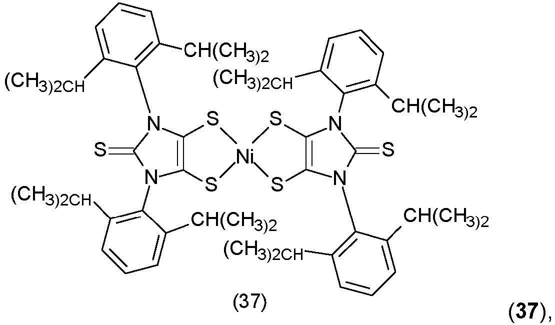

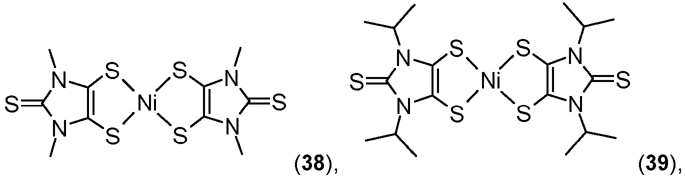

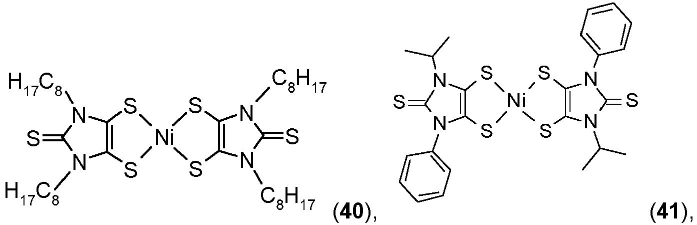

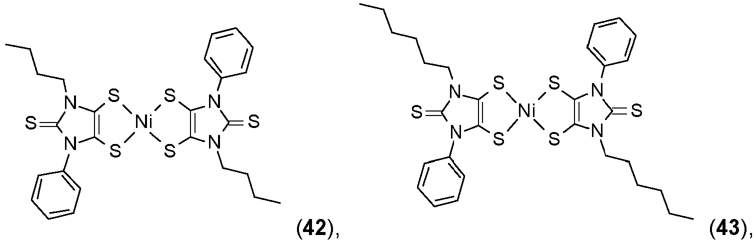

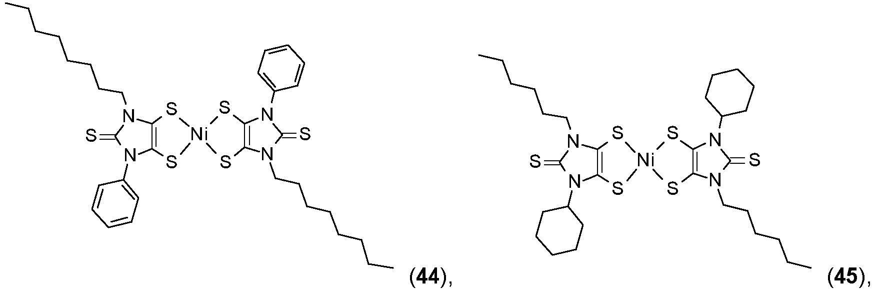

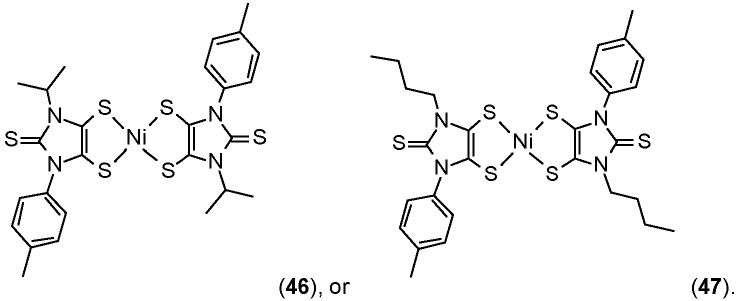

8. Use of the particles according to any of claims 1 to 7 as IR absorber for optical filter applications, for plasma display panels, for laser marking of paper or plastics, for laser welding of plastics, for 3D printing, for fuel marking, as marker for circular economic products, for the curing of surface-coatings using IR radiators, for the drying and curing of print, for the fixing of toners on paper or plastics, for heat shielding applications, for invisible and/or IR readable bar codes, or as IR absorber in security printing.

9. A printing ink formulation, comprising the particles according to any of claims 1 to 7.

10. A printing ink formulation according to claim 9, comprising
a) the particles according any of claims 1 to 7,
b) a polymeric binder,
e) a solvent,
f) optionally at least one colorant, and
e) optionally at least one further additive.

11. The printing ink formulation according to claim 9, or 10, comprising
a) 0.0001 to 25 % by weight of the particles according to any of claims 1 to 7,
b) 5 to 74 % by weight of at least one polymeric binder,
c) 1 to 94.9999 % by weight of at least one solvent,
d) 0 to 25 % by weight of at least one colorant, and
e) 0 to 25 % by weight of at least one further additive,
wherein the sum of components a) to e) adds up to 100%.

12. A process for the manufacture of a security document comprising the steps printing on a substrate a printing ink formulation according to any of claims 9 to 11.

13. A security document, comprising a substrate and at least one compound according to any of claims 1 to 7, or obtainable by the process according to claim 12.

14. The security document according to claim 13, which is selected from a bank note, a passport, a check, a voucher, an ID- or transaction card, a stamp and a tax label.

15. A process for the production of the particles according to claims 1 to 7, comprising
a) providing a mixture of a compound of formula 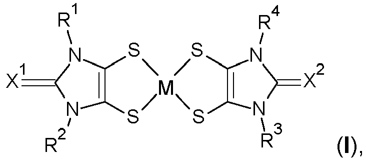 wherein
M is selected from Ni, Pd and Pt,
X¹ and X² are each independently of each other sulfur or oxygen,
R¹, R², R³, and R⁴ are independently selected from an unsubstituted, or substituted alkyl group, an unsubstituted or substituted aryl group and an unsubstituted or substituted heteroaryl group, a solvent and an inorganic salt; and
b) kneading the mixture at a temperature of from 20 to 150°C for 2 to 15 hours.

16. The process according to claim 15, wherein the inorganic salt is selected from sodium chloride and sodium sulfate.

17. The process according to claim 15, or 16, wherein the solvent is an aliphatic alcohol, diol or polyol, especially diethylene glycol, or triethylene glycol, or a mixture thereof.

## Patentansprüche

1. Partikel einer Verbindung der Formel 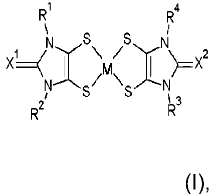 wobei
M wird aus Ni, Pd und Pt ausgewählt,
X¹ und X² sind jeweils unabhängig voneinander Schwefel oder Sauerstoff,
R¹, R², R³ und R⁴ werden unabhängig voneinander aus einer unersetzten oder substituierten Alkylgruppe, einer unsubstituierten oder substituierten Cycloalkylgruppe, einer unsubstitierten oder substituierten Arylgruppe und einer unsubstituierten oder substituierten Heteroaryl ausgewählt Gruppe,
wobei die Partikel eine mediane Partikelgröße (D₅₀) im Bereich von 30 nm bis 90 nm haben - wobei D10 größer als 20 nm ist.

2. Die Teilchen gemäß Behauptung 1, wobei die Verbindung der Formel (I) ein Com-Pfund Formel 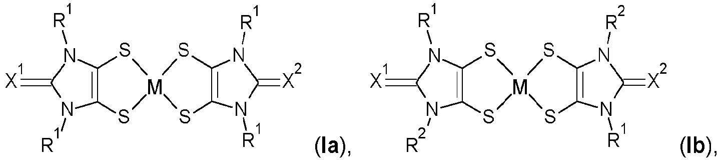 or 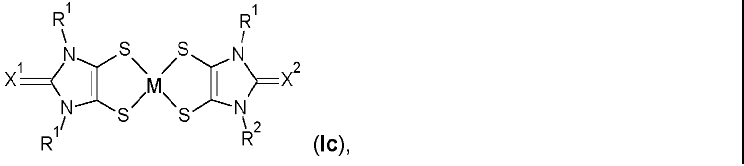 Wobei M, X1, X2, R1 in Anspruch 1 definiert sind.

3. Die Teilchen gemäß Behauptung 2, wobei in der Verbindung der Formel (la) R¹ eine unersetzte oder ersetzte Alkylgruppe eine unersetzte oder ersetzte Arylgruppe ist.

4. Die Partikel gemäß Anspruch 2, wobei in der Verbindung der Formel **(Ib),** oder **(le)** R¹ ist eine unersetzte oder ersetzte Alkylgruppe und R² eine unersetzte oder substituierte Arylgruppe.

5. Die Teilchen gemäß einem der Behauptungen 1 bis 4, wobei X¹ und X² Schwefel sind.

6. Die Teilchen gemäß jeder der Behauptungen 1 bis 5, wobei M Ni ist.

7. Die Partikel gemäß einem der Ansprüche 1 bis 6, wobei die Verbindung der (I) ist eine Zusammensetzung der Formel

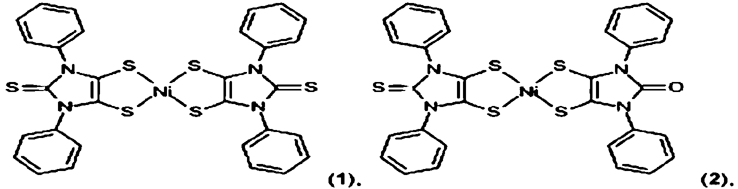

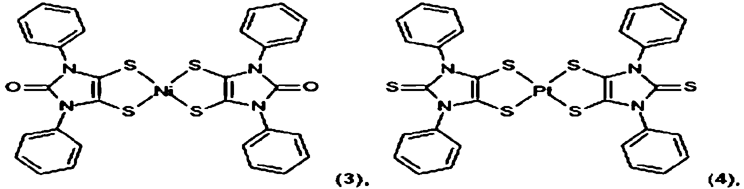

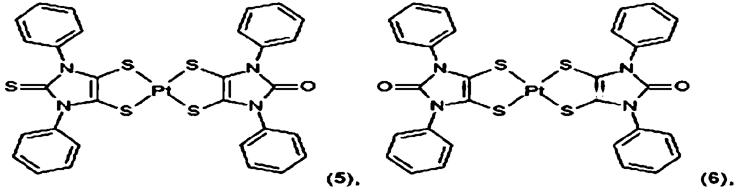

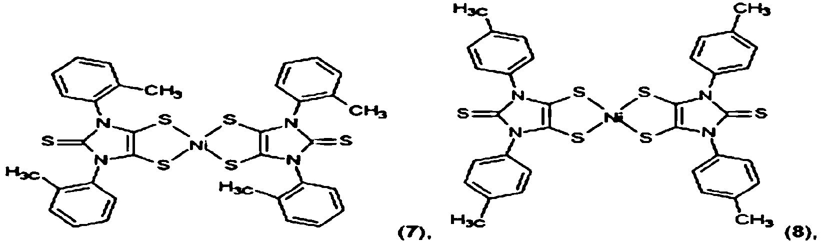

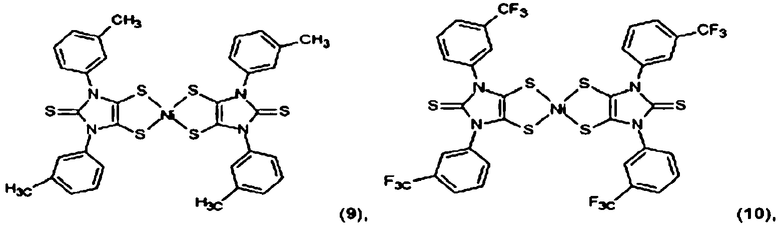

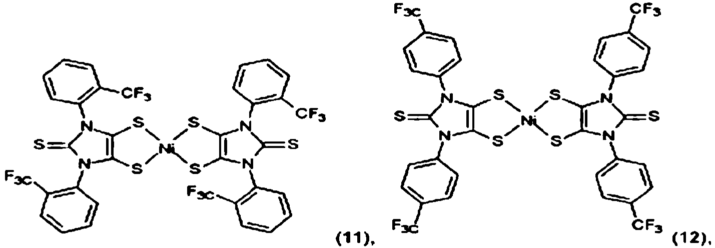

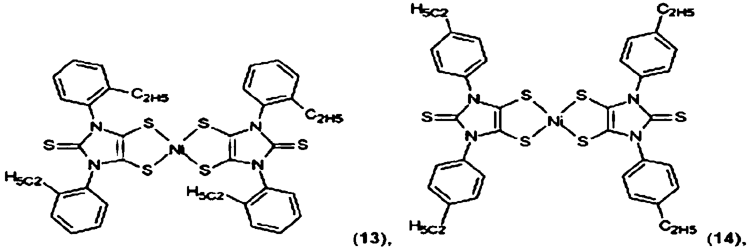

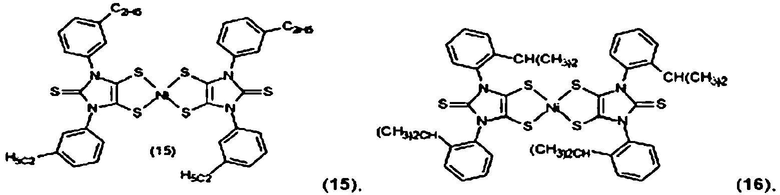

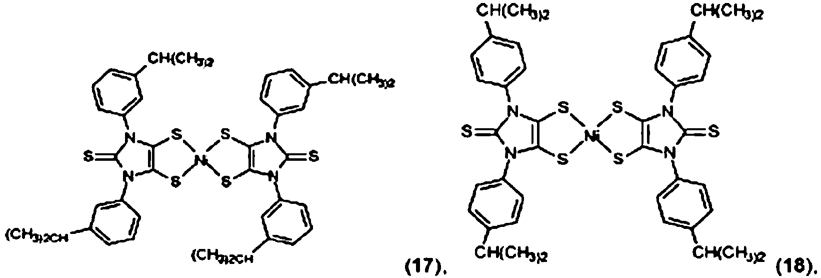

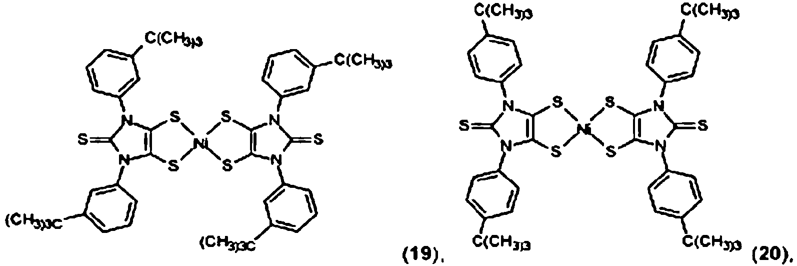

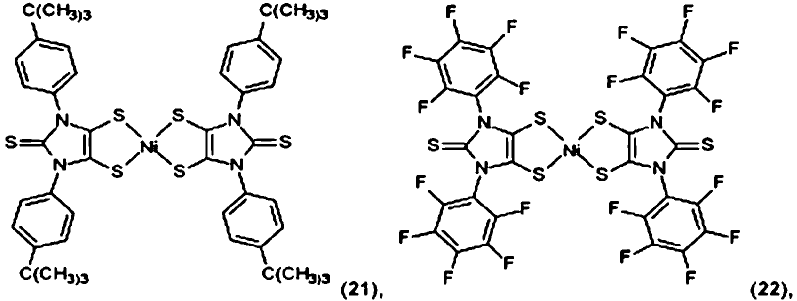

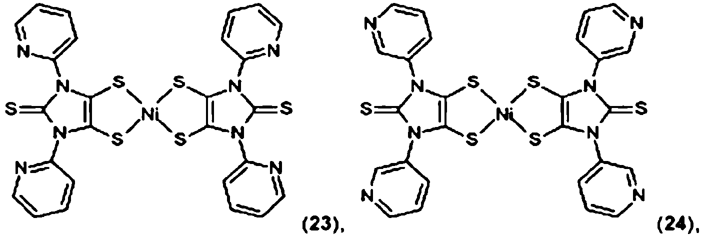

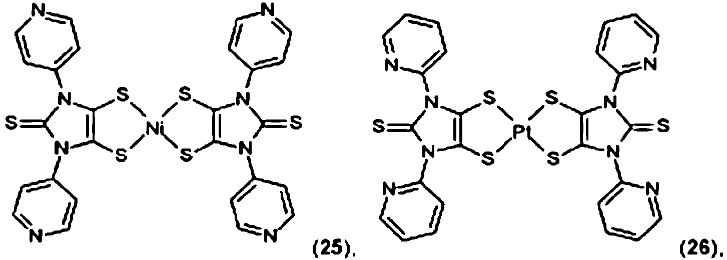

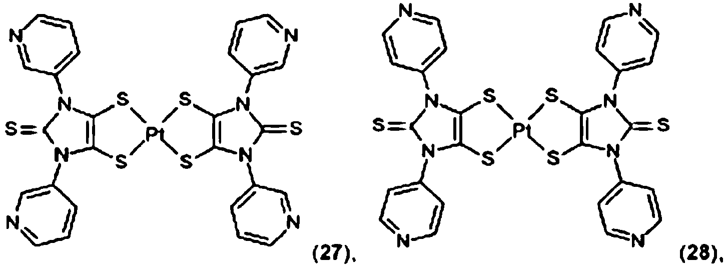

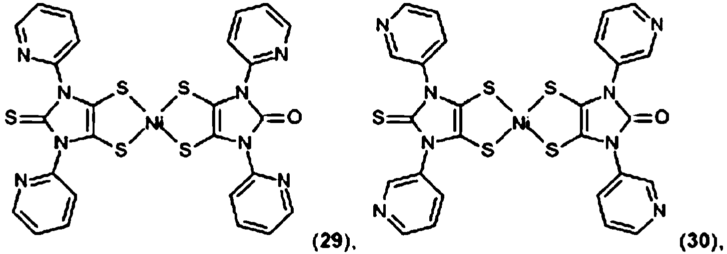

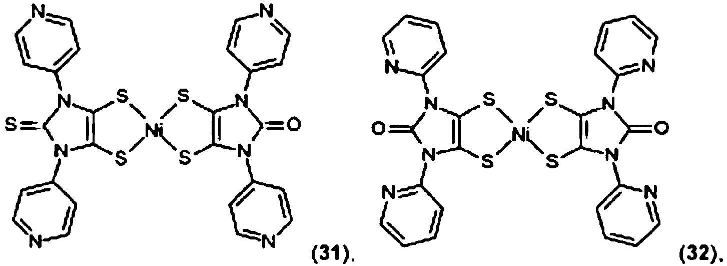

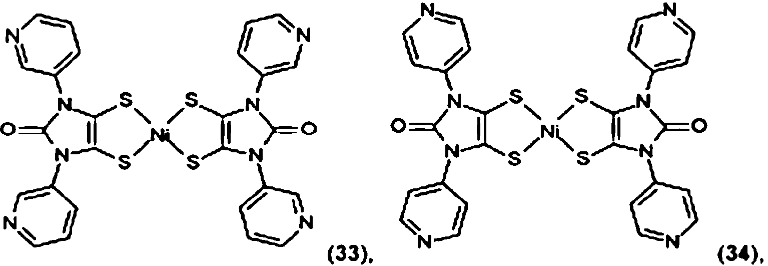

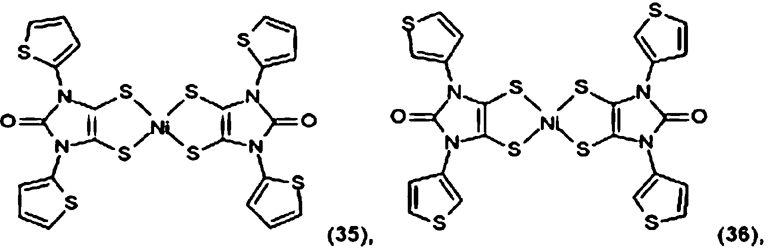

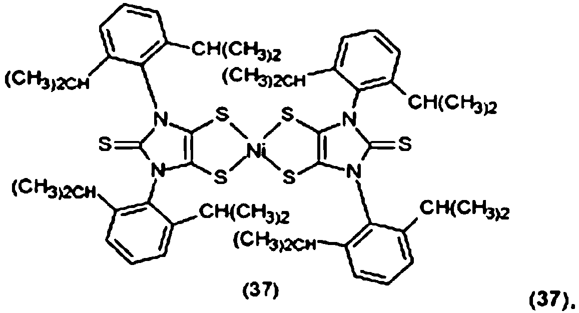

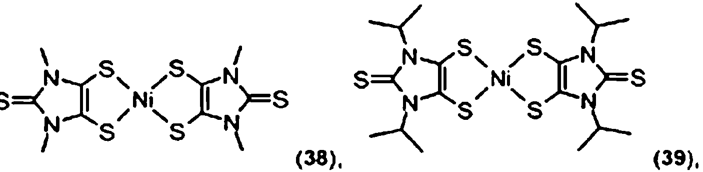

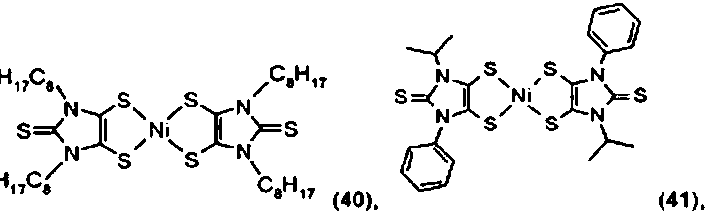

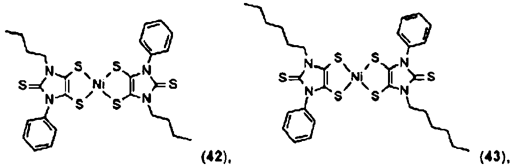

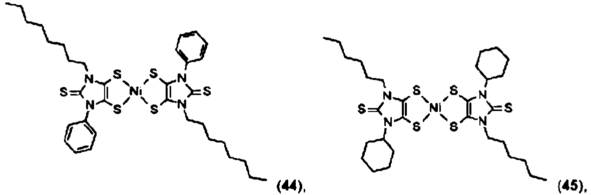

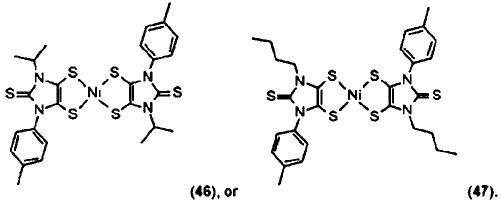

8. Verwendung der Partikel gemäß jedem der Ansprüche 1 bis 7 als IR-Absorber für Anwendungen optischer Filter, für Plasmadisplay-Panels, für Lasermarkierung von Papier oder Plastiken, für das Laserschweißen von Kunststoffen, für den 3D-Druck, für die Brennstoffmarkierung, als Markierer für zirkuelle wirtschaftliche Produkte, für das Aushärten von Oberflächenbeschichtungen mit IR-Radiatoren, für das Trocknen und Aushärten von Drucken, zum Fixieren von Tonern auf Papier oder Kunststoffen, für Hitzeschutzanwendungen für unsichtbare und/oder IR-lesbare Barcodes oder als IR-Absorber im Sicherheitsdruck.

9. Eine Druckfarbe-Formulierung, die die Partikel gemäß einem der Ansprüche 1 bis 7 umfasst.

10. Eine Druckfarbe-Formulierung gemäß Anspruch 9, bestehend aus
a) die Teilchen gemäß einer der Behauptungen 1 bis 7,
b) ein polymeres Bindemittel,
e) ein Lösungsmittel,
f) optional mindestens einen Farbstoff, und
e) optional mindestens einen weiteren Zusatzstoff.

11. Die Druckfarbe-Formulierung gemäß Anspruch 9 oder 10 umfasst
a) 0,0001 bis 25 % nach Gewicht der Partikel gemäß einer der Behauptungen 1 bis 7,
b) 5 bis 74 % nach Gewicht von mindestens einem polymeren Bindemittel,
c) 1 bis 94,9999 % nach Gewicht von mindestens einem Lösungsmittel,
d) 0 bis 25 % nach Gewicht von mindestens einem Farbstoff, und
e) 0 bis 25 % nach Gewicht von mindestens einem weiteren Zusatzstoff, wobei die Summe der Komponenten a) Toe) 100 % ergibt.

12. Ein Verfahren zur Herstellung eines Sicherheitsdokuments, das die Schritte umfasst, in denen auf einem Substrat eine Druckfarbe-Formulierung gemäß einer der Ansprüche 9 bis 11 gedruckt wird.

13. Ein Sicherheitsdokument, das ein Substrat und mindestens eine Verbindung enthält, die einem der Ansprüche 1 bis 7 entspricht oder durch das Verfahren gemäß Anspruch 12 erhältlich ist.

14. Das Sicherheitsdokument gemäß Anspruch 13, das aus einer Banknote, einem Reisepass, einem Scheck, einem Gutschein, einer Ausweis- oder Transaktionskarte, einem Stempel und einer Steuerl-Bel.

15. Ein Prozess zur Erzeugung der Partikel gemäß den Ansprüchen 1 bis 7, bestehend aus
a) wobei eine Mischung aus einer Verbindung der Formel bereitgestellt wird 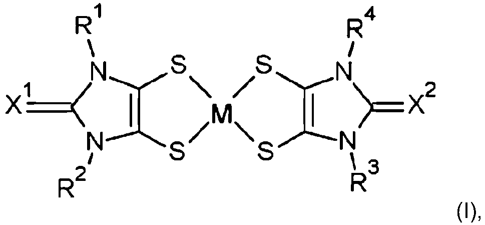 wobei
M wird aus Ni, Pd und Pt ausgewählt,
X1 und X2 sind jeweils unabhängig voneinander Schwefel oder Sauerstoff, R1, R2, R3 und R4 werden unabhängig voneinander aus einer unersetzten oder substituierten Alkylgruppe, einer unersetzten oder ersetzten Arylgruppe und einer unersetzten
oder eine heteroaryle Gruppe, ein Lösungsmittel und ein anorganisches Salz; und
b) Die Mischung wird bei einer Temperatur von 20 bis 150 °C für 2 bis 15 Stunden geknetet.

16. Das Verfahren laut Anspruch 15, bei dem das anorganische Salz aus So-Diumchlorid und Natriumsulfat ausgewählt wird.

17. Das Verfahren gemäß anspruch 15 oder 16, bei dem das lösungsmittel ein aliphatisches alco-hol, diol oder polyol ist, insbesondere diethylenglykol, triethylenglykol oder eine mischung daraus.

## Revendications

1. Particules d'un composé de la formule 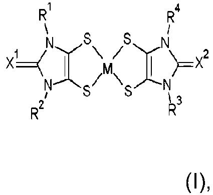 où
M est sélectionné parmi Ni, et Pt,
X¹ et X² sont chacun indépendamment du soufre ou de l'oxygène,
R¹, R², R³ et R⁴ sont sélectionnés indépendamment à partir d'un groupe alkyle non substitué ou substitué, d'un groupe cycloalkyle non substitué ou substitué, d'un groupe aryle non substitué ou substitué et d'un groupe hétéroaryle non substitué ou substitué
Groupe,
où les particules ont une taille médiane de particule (D₅₀) allant de 30 nm à 90 nm - où D10 est supérieur à 20 nm.

2. Les particules selon l'affirmation 1, où le composé de la formule (I) est un com-Formule de la livre 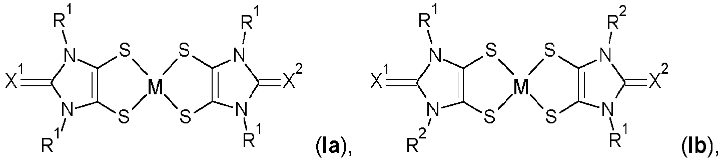 or 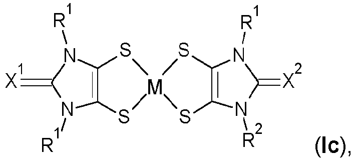 Où M, X1, X2, R1 sont définis dans la revendication 1.

3. Les particules selon la revendication 2, où le composé de la formule (la) R1, un groupe alkyle non remplacé ou remplacé, est un groupe aryle non remplacé ou substitué.

4. Les particules selon l'affirmation 2, dans laquelle dans le composé de la formule (Ib), ou
(le) R¹ est un groupe alkyle non remplacé ou substitué et R² est un groupe aryle non remplacé ou substitué.

5. Les particules selon l'une des affirmations 1 à 4, où X¹ et X² sont du soufre.

6. Les particules selon chacune des affirmations de 1 à 5, où M est Ni.

7. Les particules selon l'une des revendications 1 à 6, où le composé de la formule (I) est une composition de la formule

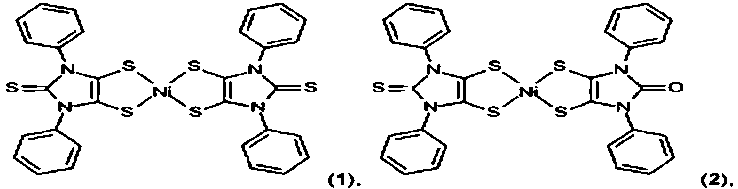

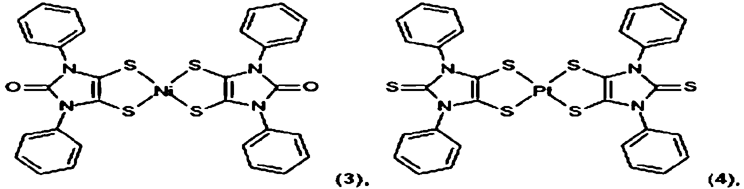

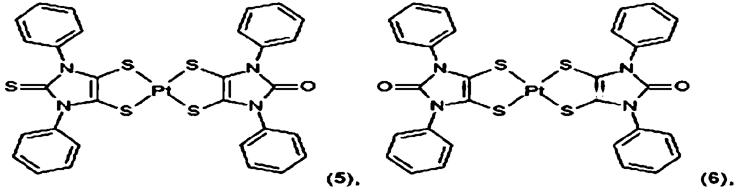

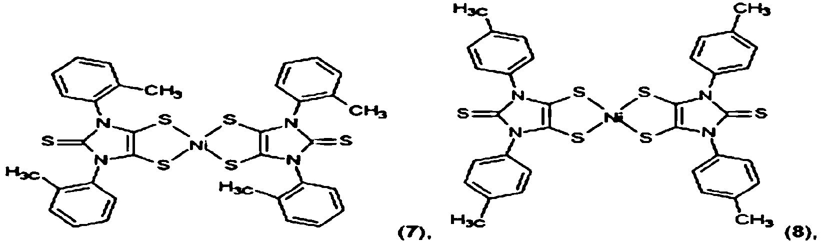

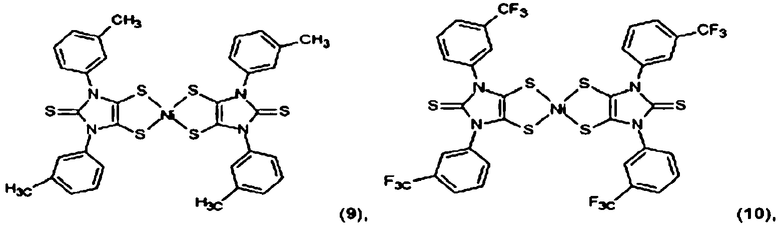

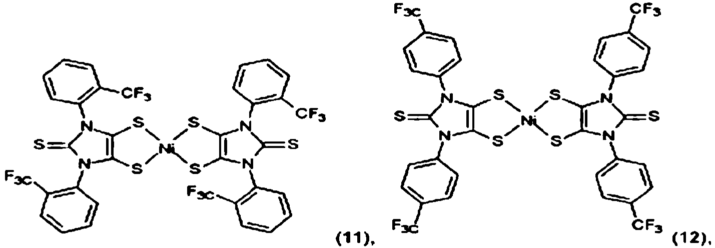

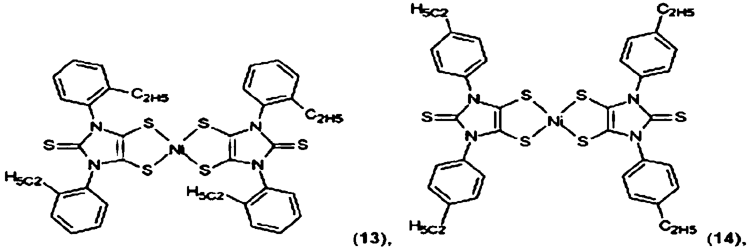

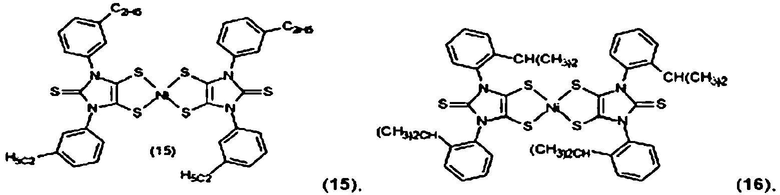

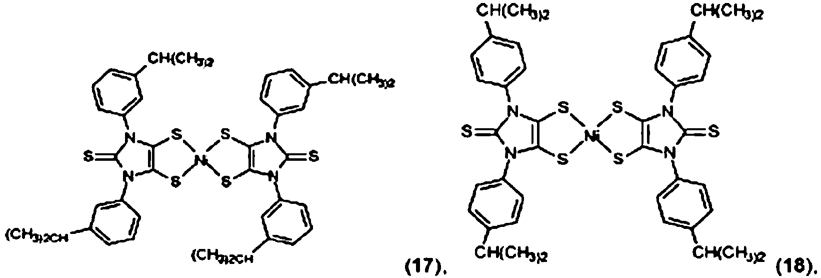

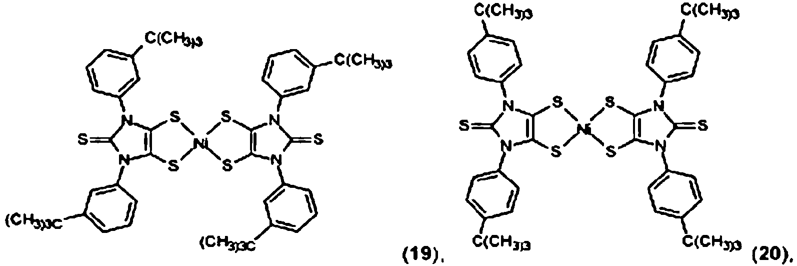

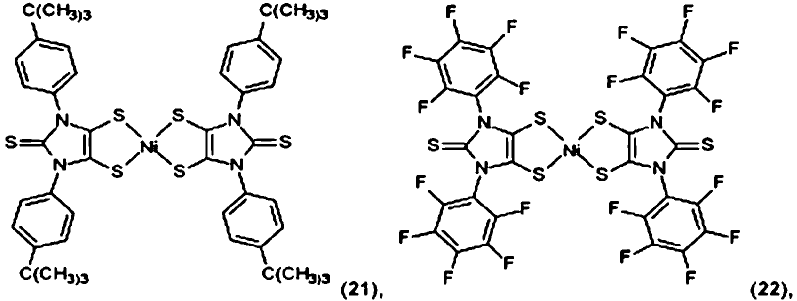

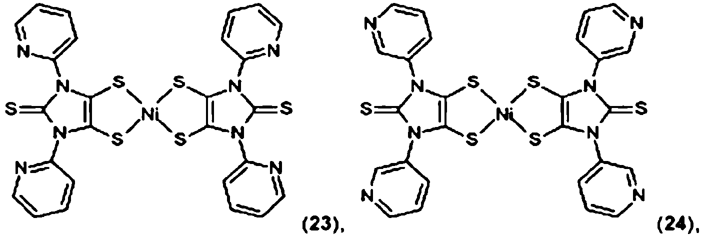

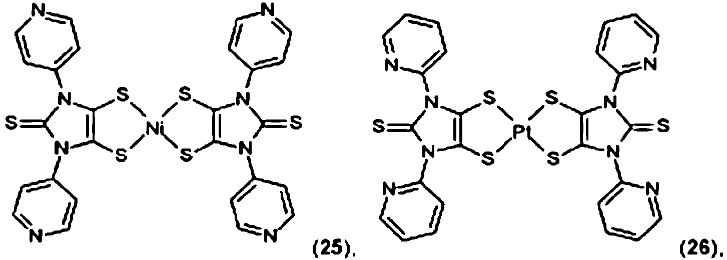

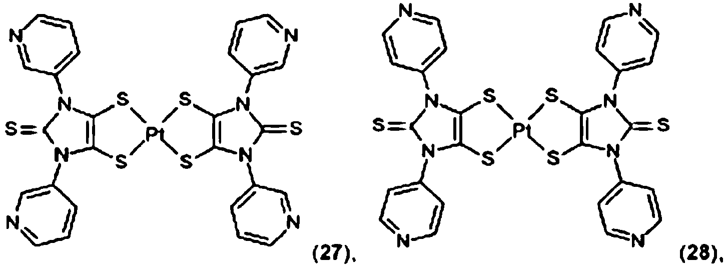

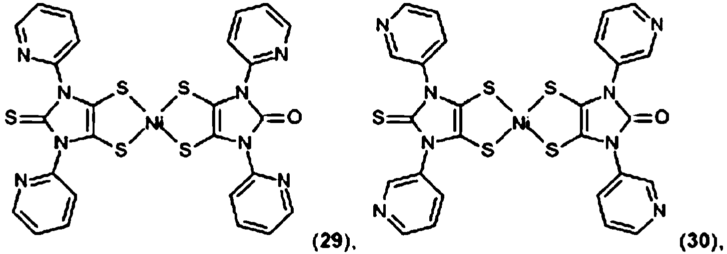

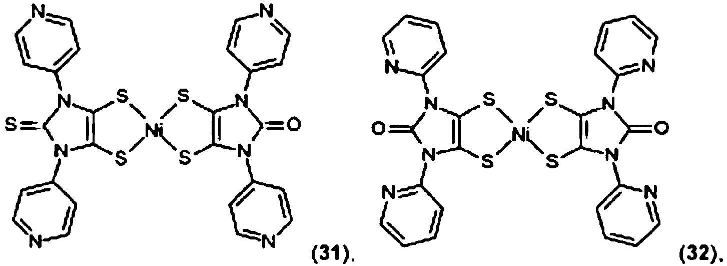

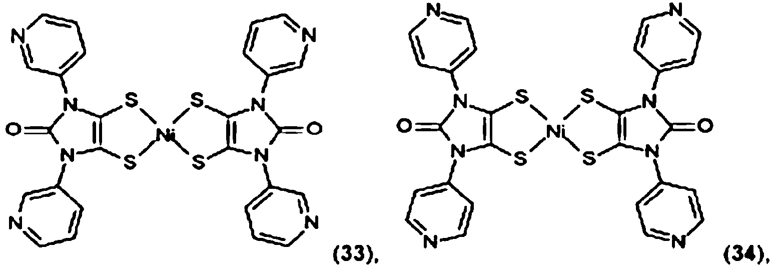

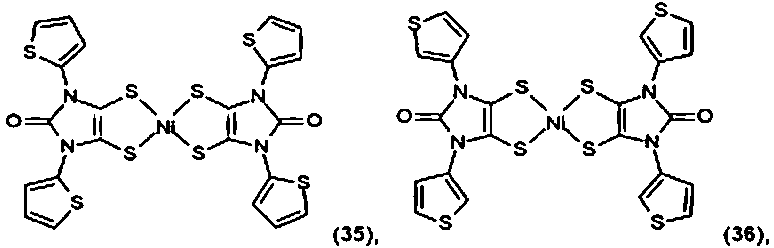

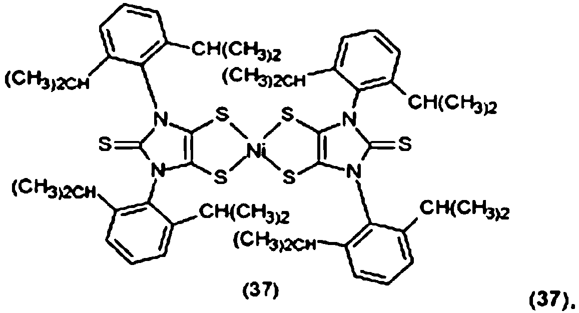

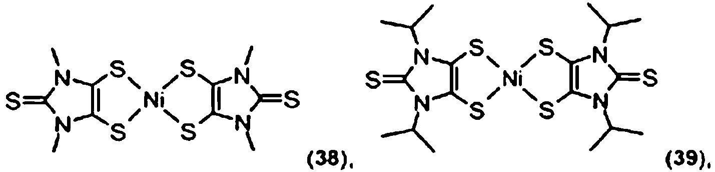

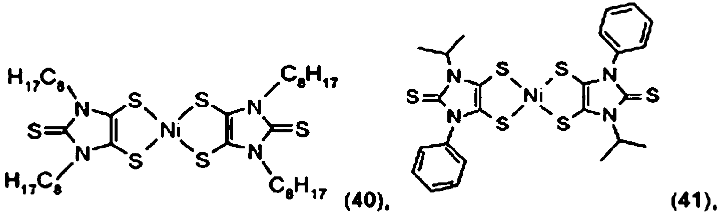

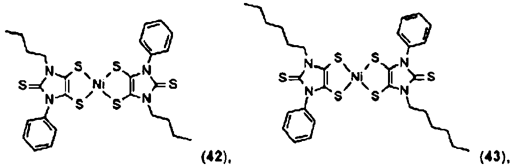

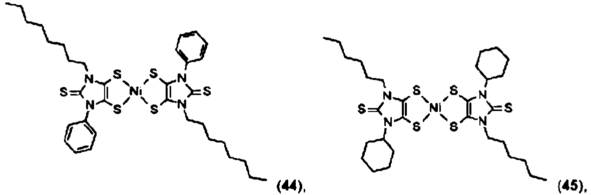

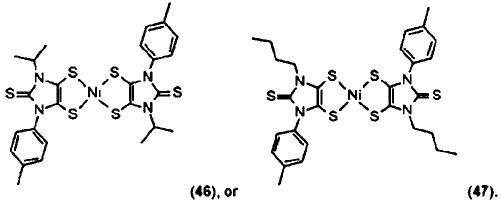

8. Utilisation des particules selon chacune des revendications 1 à 7 comme absorbeurs IR pour des applications de filtres optiques, pour des panneaux d'affichage plasma, pour le marquage laser de papier ou de plastiques, pour le soudage laser de plastiques, pour l'impression 3D, pour le marquage de carburant, comme marqueurs pour produits circulaires économiques, pour le durcissement des revêtements de surface avec des radiateurs IR, pour le séchage et le durcissement des impressions, pour la fixation de toners sur papier ou plastique, pour
Applications de protection thermique pour des codes-barres invisibles et/ou lisibles IR ou comme absorbeurs IR dans l'impression de sécurité.

9. Une formulation d'encre d'impression comprenant les particules selon l'une des revendications 1 à 7.

10. Une formulation d'encre d'impression selon la revendication 9, composée de
a) les particules selon l'une des affirmations 1 à 7,
b) un liant polymère,
e) un solvant,
f) optionnellement, au moins une teinture, et
e) Éventuellement, au moins un autre additif.

11. La formulation de l'encre d'impression selon la revendication 9 ou 10 comprend
a)0,0001 à 25 % en poids des particules selon l'une des affirmations 1 jusqu'à 7,
b) 5 à 74 % en poids d'au moins un liant polymérique,
c) 1 à 94,9999 % en poids d'au moins un solvant,
d) 0 à 25 % en poids d'au moins un colorant, et
e) 0 à 25 % en poids d'au moins un autre additif,
où la somme des composantes a) est de 100 %.

12. Une méthode de préparation d'un document de sécurité comprenant les étapes durant lesquelles une formulation d'encre d'impression selon l'une des revendications 9 à 11 est imprimée sur un substrat.

13. Un document de sécurité contenant un substrat et au moins un composé conforme à l'une des revendications 1 à 7 ou obtenu par la méthode de l'affirmation 12.

14. Le document de garantie selon la revendication 13, composé d'un billet de banque, passeport, chèque, bon, carte d'identité ou carte de transaction, tampon et ticket fiscal, Bel.

15. Un processus de génération des particules selon les revendications 1 à 7, composé de
a) où un mélange d'un composé de la formule est fourni 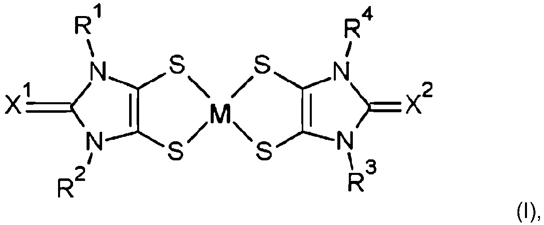 où
M est sélectionné parmi Ni, et Pt,
X1 et X2 sont chacun indépendamment du soufre ou de l'oxygène, R1, R2, R3 et R4 sont indépendamment composés d'un groupe alkyle non remplacé ou substitut, d'un groupe aryl non remplacé ou substitué et d'un groupe alkyle non remplacé
ou un groupe hétéroaryle, un solvant et un sel inorganique ; et
b) Le mélange est pétri à une température de 20 à 150 °C pendant 2 à 15 heures.

16. La méthode de la revendication 15 consiste à sélectionner le sel inorganique à partir de chlorure de sodium et de sulfate de sodium.

17. La méthode de la revendication 15 ou 16 consiste à utiliser le solvant comme un alco-hol, diol ou polyol aliphatique, en particulier le diéthylène glycol, le triéthylène glycol ou un mélange de ceux-ci.
